# EUROPEAN PATENT APPLICATION

(11) **EP 4 296 674 A1**
(43) Date of publication of application: **27.12.2023**
(21) Application number: 22305894.2
(22) Date of filing: 20.06.2022
(51) Int. Cl.: G01N 33/569, C12N 15/74

(54) **INNOVATIVE MOLECULES DECREASING VIRULENCE OF MYCOBACTERIUM FOR THE TREATMENT OF TUBERCULOSIS**

(71) Applicant: Université Toulouse III - Paul Sabatier, 31400 Toulouse (FR); Centre national de la recherche scientifique, 75016 Paris (FR); Institut National de la Santé et de la Recherche Médicale (INSERM), 75013 Paris (FR); Université d'Aix Marseille, 13007 Marseille 7 (FR); Institut Jean Paoli & Irène Calmettes, 13009 Marseille (FR)
(72) Inventor: QUEMARD, Annaïk, 31077 Toulouse Cedex 04 (FR); BARDOU, Fabienne, 31077 Toulouse Cedex 04 (FR); BORIES, Pascaline, 31077 Toulouse Cedex 04 (FR); DUCOUX-PETIT, Manuelle, 31077 Toulouse Cedex 04 (FR); ROCHE, Philippe, 13273 Marseille Cedex 09 (FR); CONSTANT, Patricia, 31077 Toulouse cedex 04 (FR); TRANIER, Samuel, 31077 Toulouse Cedex 04 (FR); BON, Cécile, 31077 Toulouse Cedex 04 (FR); MOUREY, Lionel, 31077 Toulouse Cedex 04 (FR); MARCOUX, Julien, 31077 Toulouse cedex 04 (FR)
(74) Representative: Cabinet Becker et Associés

(57) **Abstract**

The present invention relates to a novel strategy for the treatment of tuberculosis based on molecules inhibiting the interaction between HadB and HadD proteins of *Mycobacterium tuberculosis* and to a novel screening method for selecting these molecules.

## Description

### FIELD OF THE INVENTION

The present invention relates to the medicine, in particular to the treatment of tuberculosis, especially by decreasing the virulence of *Mycobacterium tuberculosis.*

### BACKGROUND OF THE INVENTION

Despite the availability of a vaccine and an antibiotherapy together with an undeniable improvement in patient care, tuberculosis (TB) remains today a major public health concern. Indeed, with the exception of COVID-19, it is the leading cause of death due to a single infectious agent in the world, triggering each year about 10 million new cases and 1.5 million deaths (WHO, Global tuberculosis report 2020). The lack of control of the disease is largely due to the development of multi- and extremely-drug resistance of *Mycobacterium tuberculosis (Mtb),* the causative agent, making the design of new therapeutic strategies very urgent (WHO, Global tuberculosis report 2020). Targeting proteins required for *Mtb* virulence instead of targeting essential enzymes constitutes an unconventional and attractive strategy to develop novel TB drugs because the occurrence of resistance is expected to be significantly reduced, the bacterial essential physiological processes being not directly impacted. Moreover, it would allow circumventing the problem of cross-resistance with standard treatments that target essential enzyme catalytic sites. Furthermore, protein-protein interactions (PPIs) play a pivotal role in a myriad of cellular processes and have emerged as an invaluable reservoir of therapeutic targets of a new kind [(Carro, L. Beilstein journal of organic chemistry 14, 2881-2896 (2018)]. Thus, designing PPI modulators has successfully led to novel treatments (mainly for cancer therapy so far) recently approved or in clinical validation, and shows great promise for the discovery of new antibiotics, including against TB [Stefan, M.A., et al. Sci Rep 10, 21309 (2020); Kling, A., et al. Science 348, 1106-1112 (2015)].

*Mtb* synthesizes a variety of atypical lipids that contribute to the complexity and very low permeability of its cell envelope. They include the mycolate-containing compounds, which constitute the major components of the external membrane, the so-called "mycomembrane". Mycolic acids (MAs) are covalently linked either to the arabinogalactan or to diverse polyol molecules such as trehalose. These lipids are essential for the bacterial viability but they also play a determinant role in *Mtb* virulence and persistence by modulating the immune response of the infected host. Therefore, MA biosynthesis represents one of the Achilles' heels of *Mtb.* This is particularly true for the Fatty Acid Synthase type II (FAS-II) system, which is targeted by anti-TB drugs such as isoniazid, ethionamide and thiacetazone as well as promising drug candidates (Reviews: Nataraj, V. et al. Mol. Microbiol. 98, 7-16 (2015). Bahuguna, A. & Rawat, D. S. Medicinal Research Reviews 40, 263-292 (2020)). MAs are high molecular weight (up to 100 carbon atoms) α-branched and β-hydroxylated fatty acids that hold different types of chemical modifications at two specific positions. Three main classes coexist in *Mtb,* α*-,* methoxy-, and keto-MAs. Their complex biosynthesis pathway involves several multienzyme systems, including two fatty acid synthases, FAS-I and FAS-II. FAS-I corresponds to a single multi-domain enzyme, while FAS-II is composed of acyl carrier protein (ACP)-dependent discrete mono-functional enzymes. Both systems process iteratively to condense a malonyl unit with the growing acyl chain, followed by three reaction steps (β-ketoacyl reduction, (3R)-hydroxyacyl dehydration and trans-2-enoyl reduction) leading to an increment of two carbons per elongation cycle. FAS-I provides the C₁₆-C₁₈ precursors that next enter the mycobacterial FAS-II complex. The latter is unique because it does not perform *de novo* synthesis like the classical bacterial and plant FAS-II systems, but elongates long fatty acyl chains into extremely long ones (C₆₀-C₇₀), called the meromycoloyl chains.

The third reaction step of fatty acid biosynthesis cycles is performed by (3R)-hydroxyacyl dehydratase (HAD) enzymes/domains. *Mtb* FAS-II system possesses two (3R)-hydroxyacyl-ACP dehydratases, HadAB and HadBC, which belong to the hydratase 2 subfamily and display original structures and substrate specificities with respect to other bacterial HADs. HadAB and HadBC are heterotetramers composed of the protomers HadA, HadB and HadC, encoded by a single gene cluster. An additional HAD protomer, HadD, whose gene lies upstream of the gene coding for the cyclopropane synthase CmaA2 in *Mtb* (dedicated to keto- and methoxy-MAs), has been recently identified (Lefebvre et al, 2018, Sci Rep 8(1): 6034). It was shown that HadAB mediates in the early FAS-II elongation cycles common to all MA types, whereas HadBC and HadD are involved in the late elongation cycles required for the biosynthesis of the oxygenated MAs in *Mtb.* HadBC is preferentially dedicated to the methoxy-MA pathway and HadD to that of the keto-MAs, although their function would partially overlap. A hadABC knockout mutant in *Mtb* proved to be not viable, and hadB gene was shown to be essential for M. *smegmatis* survival in axenic culture, in contrast to hadC (Lefebvre et al, 2020, Sci Rep 10(1): 2112)) and hadD genes (Lefebvre et al, 2018, *supra*) in *Mtb.* Yet, hadD gene deletion alters the MA profile, in particular the quantity and structure of keto-MAs, deeply impacting *Mtb* physiology and virulence (Lefebvre et al, 2018, Sci Rep 8(1): 6034). Thus, HadD protein is important for the fitness and the capacities of *Mtb* bacilli to assemble into colonies or biofilms, and for their tolerance to low temperature. Moreover, a marked decrease of the bacterial loads was observed in the lungs and spleen of mice infected with *Mtb* hadD deletion mutant (Lefebvre et al, 2018, Sci Rep 8(1): 6034). This is in agreement with the reported role of oxygenated MAs, and particularly keto-MAs, in the virulence of *Mtb* in macrophages and in mice as well as in tackling the host immune system.

In conclusion, tuberculosis control is notably compromised by the emergence of multidrug-resistant *Mycobacterium tuberculosis (Mtb)* bacilli. Therefore, there is an urgent need of identifying and developing new molecules for the treatment of tuberculosis targeting the virulence.

### SUMMARY OF THE INVENTION

The (3R)-hydroxyacyl dehydratase (HAD) protein HadD from the fatty acid synthase-II system is mainly dedicated to the production of keto-mycolic acids, which play a determinant role in *Mtb* virulence. More particularly, the inventors identified that HadD constitutes a valuable target for a therapeutic anti-virulence strategy. They demonstrated the existence of tight physical interactions between HadD and HadB, generating a heterotetramer. The resulting HadBD enzyme displays a HAD activity, which is lost after mutating the putative catalytic His residue in HadB active site, designating HadB as the catalytic subunit. The crystal structure of *Mtb* HadBD and HadBD-substrate analog interaction models confirms that HadD plays the role of substrate-binding subunit and provides important insights on its substrate specificity. Knowledge of the functional and structural properties of HadBD constitutes a solid basis for designing innovative anti-*Mtb* virulence molecules using HadBD, and more specifically the protein-protein interactions of this protein complex, as a pharmaceutical target. This target allows circumventing the problem of cross-resistance with standard treatments that target essential enzyme catalytic sites. In addition, the occurrence of resistance is expected to be significantly reduced, since bacterial essential physiological processes is directly impacted, and also because the amino acid residues at the HadBD interfaces are highly conserved during evolution and mutate more slowly than residues at non-binding surfaces.

Accordingly, the present invention relates to a molecule inhibiting the interaction between HadB protein of *Mtb* and HadD protein of *Mtb* or a pharmaceutical composition comprising said molecule for use in the treatment of tuberculosis, in particular by decreasing the *Mtb* virulence.

The molecule inhibiting the interaction between HadB protein of *Mtb* and HadD protein of *Mtb* can be selected from the group consisting of peptides or polypeptides or peptide mimetics, antibodies, fragments or derivatives thereof such as nanobodies, aptamers, Spiegelmers, and chemical compounds, preferably chemical compounds.

In a particular aspect, the molecule binds to HadD protein of *Mycobacterium tuberculosis* at one or several amino acid residues in position F21, D37, H39, T41, H42, A51, Y53, V57, A58, P59, L60, T61, F62, L63, A64, 165, A66, G67, R68, R69, V70, Q71, L72, 174, F75, N79, 180, P81, 182, N83, 184, V87, H89, Q92, F94, F96, R98, P99, 1100, F108, T110, 1126, R127, S128, V130, V138, V139, S141, V142, V143, M145, A150, H151 of HadD of *Mtb,* the positions being as indicated in SEQ ID NO: 2.

In another particular aspect, the molecule binds to HadD of *Mycobacterium tuberculosis* at one or several amino acid residues as defined in the following groups, the positions being as indicated in SEQ ID NO: 2:
- T61, L63, A64, G67, R68, V70, Q71, L72, H89, F75, 184, V87, H89, Q92, F94, F108, S128, V130, S141, V143 and M145;
- D37, H39, T41, H42, A51, Y53, V57, A58, P59, L60, F62, L63, F94, F96, R98, P99, 1100, V138 and V139;
- L63, A64, 165, G67, R68, V70, Q71, H89, Q92, F94, F108, T110, 1126, R127, S128, V130, S141, V142 and V143;
- R68, Q71, L72, 174, F75, 184, V87, H89, V143 and M145;
- D37, H39, T41, H42, A51, Y53, V57, A58, P59 and L60;
- F21, 165, A66, G67, R68 and R69;
- H42, V57, A58, P59, L60, F96, P99 and 1100;
- V57, A58, P59, L60, T61, F62, L63, F96, R98, P99 and 1100;
- L63, A64, G67, R69, Q71, H89, Q92, V143 and M145; and
- F75, N79, 180, P81, 182, N83, 184, A150 and H151.

Indeed, by two different *in silico* methods, these pockets have been predicted as being highly druggable.

In a particular aspect, the molecule inhibiting the interaction between HadB protein of *Mtb* and HadD protein of *Mtb* is to be used in combination with another therapeutic agent treating tuberculosis. Preferably, the therapeutic agent is an antibiotic, preferably selected from the group consisting of streptomycin, 4-aminosalicylic acid, isoniazid, pyrazinamide, cycloserine, kanamycin, ethionamide, ethambutol, capreamycin, rifampicin, bedaquiline, delamanid, pretomanid, linezolid, moxifloxacin, levofloxacin, prothiaxamine, amikacin and any combination thereof. In a specific aspect, the therapeutic agent is isoniazid and/or rifampicin.

The present invention further relates to a pharmaceutical composition comprising a molecule inhibiting the interaction between HadB protein of *Mtb* and HadD protein of *Mtb,* and optionally a pharmaceutically acceptable carrier or excipient, and to a molecule inhibiting the interaction between HadB protein of *Mtb* and HadD protein of *Mtb* for use as a drug. The pharmaceutical composition can further comprise another therapeutic agent treating tuberculosis, especially an agent as detailed herein.

The present invention further relates to a method for identifying a molecule inhibiting the interaction between HadB protein of *Mycobacterium tuberculosis* and HadD protein of *Mycobacterium tuberculosis,* wherein the method comprises determining the effect of a candidate molecule on the interaction between HadB protein and HadD protein of *Mycobacterium tuberculosis,* and selecting the candidate molecule if the interaction between HadB protein and HadD protein is decreased or prevented.

Another problem is that classical target-based high throughput screening (HTS) approach showed very limited efficacy in the tuberculosis field. It does not necessarily translate into activity on intact bacteria notably because of issues of bacterial drug uptake (due to the extremely low permeability of the mycobacterial envelope), target accessibility and drug counteraction by bacteria. Therefore, the inventors developed a new screening method based on mixed target-based whole-cell assays. This strategy allows to discover molecules that i) pass through the permeability barrier of the mycobacterial envelope, ii) are not quickly degraded or eliminated via efflux by bacteria, and iii) directly inhibit the protein-protein interaction with the target complex in its native state and physiological environment.

The present invention relates to a kit comprising one or several Mycobacterium strains and/or vectors encoding the fusion proteins necessary or useful for the screening methods and to methods for identifying a molecule inhibiting the interaction between HadB protein of *Mtb* and HadD protein of *Mtb.*

More particularly, the present invention relates to a mycobacterial tryptophan auxotrophic strain comprising an expression cassette encoding a HadB protein of *Mycobacterium tuberculosis* fused to a N-terminal fragment of *Saccharomyces cerevisiae* Trplp protein (Ntrp) and an expression cassette encoding a HadD protein of *Mycobacterium tuberculosis* fused a C-terminal fragment of *Saccharomyces cerevisiae* Trplp protein (Ctrp), thereby providing a mycobacterial tryptophan auxotrophic strain expressing a fusion protein of HadB protein of *Mycobacterium tuberculosis* and the Ntrp fragment of *Saccharomyces cerevisiae* Trplp protein and a fusion protein of HadD protein of *Mycobacterium tuberculosis* and the Ctrp fragment of *Saccharomyces cerevisiae* Trplp protein.

In addition, the present invention relates to a kit comprising 1) the mycobacterial tryptophan auxotrophic strain as defined herein comprising an expression cassette encoding a HadB protein of *Mycobacterium tuberculosis* fused to a Ntrp fragment of *Saccharomyces cerevisiae* Trplp protein and an expression cassette encoding a HadD protein of *Mycobacterium tuberculosis* fused a Ctrp fragment of *Saccharomyces cerevisiae* Trplp protein, thereby providing a mycobacterial tryptophan auxotrophic strain expressing a fusion protein of HadB protein of *Mycobacterium tuberculosis* and the Ntrp fragment of Trplp protein and a fusion protein of HadD protein of *Mycobacterium tuberculosis* and the Ctrp fragment of Trplp protein, and 2) a mycobacterial tryptophan auxotrophic strain comprising an expression cassette encoding a fusion protein comprising the Ntrp fragment of *Saccharomyces cerevisiae* Trplp protein fused to Esat6 and an expression cassette encoding a fusion comprising the Ctrp fragment of *Saccharomyces cerevisiae* Trp1p protein fused to Cfp10. Optionally, the kit can further comprise a mycobacterial tryptophan auxotrophic strain comprising an expression cassette encoding a fusion protein comprising the Ntrp fragment of *Saccharomyces cerevisiae* Trplp protein fused to HadB protein and an expression cassette encoding a fusion protein comprising the Ctrp fragment of *Saccharomyces cerevisiae* Trplp protein fused to Cfp10; and/or a mycobacterial tryptophan auxotrophic strain comprising an expression cassette encoding a fusion protein comprising the Ntrp fragment of *Saccharomyces cerevisiae* Trplp protein fused to Esat6 of *Mycobacterium tuberculosis* and an expression cassette encoding a fusion protein comprising the Ctrp fragment of *Saccharomyces cerevisiae* Trplp protein fused to HadD protein of *Mycobacterium tuberculosis.*

In a specific aspect, the present invention relates to a method for identifying a molecule inhibiting the interaction between HadB protein of *Mycobacterium tuberculosis* and HadD protein of *Mycobacterium tuberculosis,* wherein the method comprises
- contacting a strain comprising an expression cassette encoding a HadB protein of *Mycobacterium tuberculosis* fused to a Ntrp fragment of *Saccharomyces cerevisiae* Trplp protein and an expression cassette encoding a HadD protein of *Mycobacterium tuberculosis* fused a Ctrp fragment of *Saccharomyces cerevisiae* Trplp protein with a candidate molecule in absence of tryptophan,
- measuring the growth of said strain in absence of tryptophan; and
- selecting the candidate molecule as inhibitor of the interaction between HadB protein of *Mycobacterium tuberculosis* and HadD protein of *Mycobacterium tuberculosis* if the strain contacted with the candidate molecule has a reduced growth in absence of tryptophan compared to the growth of the strain in absence of the candidate molecule and of tryptophan.

Optionally, the method further comprises
- contacting a strain comprising an expression cassette encoding a HadB protein of *Mycobacterium tuberculosis* fused to a Ntrp fragment of *Saccharomyces cerevisiae* Trplp protein and an expression cassette encoding a HadD protein of *Mycobacterium tuberculosis* fused a Ctrp fragment of *Saccharomyces cerevisiae* Trplp protein with a candidate molecule in presence and in absence of tryptophan,
- measuring the growth of said strain in presence and in absence of tryptophan; and
- selecting the candidate molecule as inhibitor of the interaction between HadB protein of *Mycobacterium tuberculosis* and HadD protein of *Mycobacterium tuberculosis* if the strain contacted with the candidate molecule has a reduced growth of the strain in absence of tryptophan compared to the growth of the strain in presence of both the candidate molecule and tryptophan.

In addition and optionally, the method further comprises
an assay with a mycobacterial tryptophan auxotrophic strain comprising an expression cassette encoding a fusion comprising the Ntrp fragment of *Saccharomyces cerevisiae* Trplp protein fused to Esat6 and an expression cassette encoding a fusion comprising the Ctrp fragment of *Saccharomyces cerevisiae* Trplp protein fused to Cfp10; the assay comprising contacting said strain with a candidate molecule in absence of tryptophane, measuring the strain growth, and selecting the candidate molecule if the growth inhibition of the strain (Ntrp-hadB/hadD-Ctrp) in absence of tryptophan and in presence of the candidate molecule is higher than the growth inhibition of the strain (Ntrp-Esat6 / CfplO-Ctrp) in presence of the candidate molecule and in absence of tryptophan.

Preferably, the method is a high throughput method and the method is carried in parallel with more than 100, 500, 1000, 1500, 5000 candidate molecules.

This method has been used to carry out the screening of a chemical library including about 10,000 compounds and results in the selection of no less than 137 compounds capable of inhibiting the interaction between HadB of *Mycobacterium tuberculosis* and HadD of *Mycobacterium tuberculosis* (see Example 3).

### BRIEF DESCRIPTIONS OF THE FIGURES

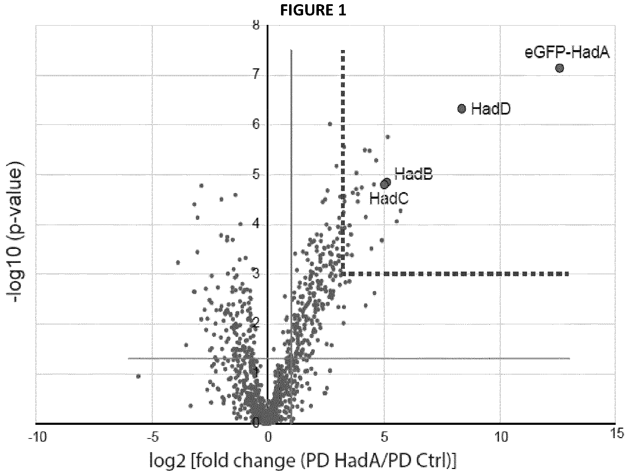

**Figure 1****. HadD makes strong specific interactions with HadAB from FAS-II in M. *bovis* BCG.** Proteomics analysis of pull-down fractions using eGFP-HadA*_{Mtb}* as a bait. Volcano plot showing enriched proteins in eGFP-HadA-based pull-down (PD HadA) versus control pull-down (PD Ctrl; using simple eGFP as a bait). Statistical analysis was performed from four independent biological replicates by two-sided Student t-test in Proline 2.1. Significantly enriched proteins proteins [fold change (PD HadA/PD Ctrl) = 2; p-value = 0.05] are located in the right upper panel, beyond the grey threshold lines. Highly enriched proteins [fold change (PD HadA/PD Ctrl) = 10; p-value = 0.001] are located beyond the magenta threshold dotted lines. The bait, GFP-HadA, and enriched copurifying HAD proteins (HadB, HadC and HadD) are colored in red.

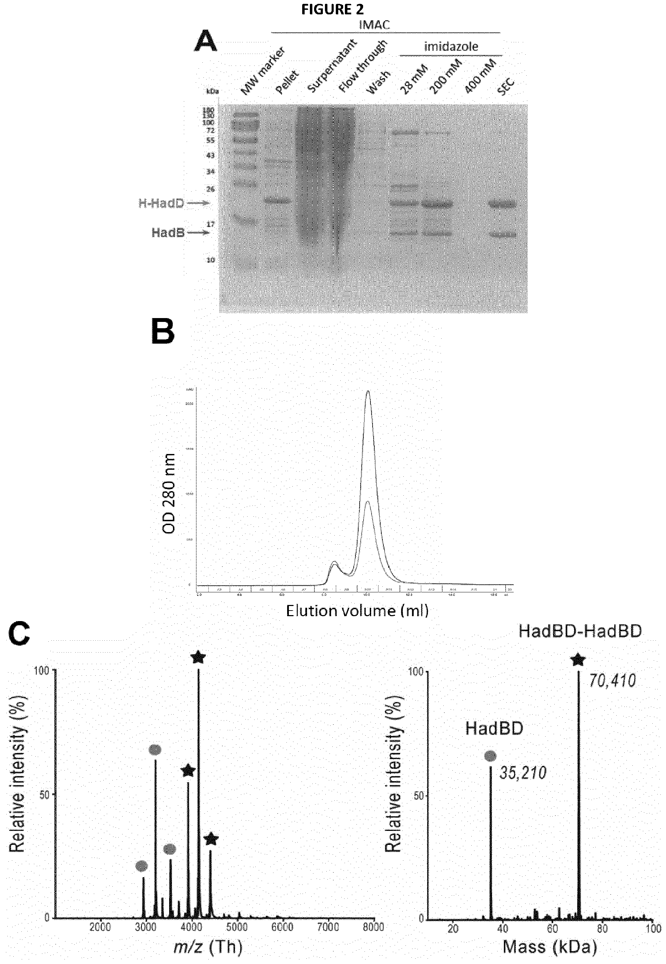

**Figure 2****. HadD*_{Mtb}* and HadB*_{Mtb}* assemble in heterocomplexes in recombinant *E. coli.*** Analyses of cobalt-based IMAC and SEC purification fractions from an *E. coli* recombinant strain coproducing H-HadD*_{Mtb}* and HadB*_{Mtb}* proteins. **A.** SDS-PAGE. The 15% acrylamide gel was stained using Coomassie Brillant blue. **B.** SEC analysis. **C.** Native MS. Left panel, multicharged spectrum; right panel, deconvoluted spectrum. Theoritical masses of (HadB/H-HadD)₂ heterotetramer (black stars): 70,390 Da (with the four N-terminal Met residues cleaved) and of HadB/H-HadD heterodimer (gray circles): 35,195 Da (with both N-terminal Met residues cleaved).

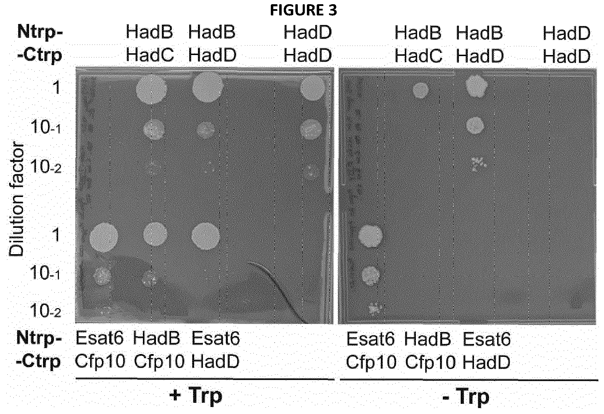

**Figure 3****. Strong interactions between HadD*_{Mtb}* and HadB*_{Mtb}* in mycobacterial** cells. Split-Trp assays in *M. smegmatis.* Recombinant *M. smegmatis* Δ*hisA* producing pairs of *Mtb* proteins fused either to the N- or C-terminal fragment of *S*. *cerevisiae* Trplp protein (Ntrp, Ctrp) were diluted 1, 10 and 100-fold in water and spotted in parallel onto minimal and Trp-supplemented media. Tested protein pairs were: Ntrp-HadB*_{Mtb}*/HadD*_{Mtb}*-Ctrp and Ntrp-HadD*_{Mtb}*/HadD*_{Mtb}*-Ctrp. Ntrp-Esat6/Cfp10-Ctrp and Ntrp-HadB/HadC-Ctrp protein pairs were used as positive controls (both HadB/HadC and the early secreted T-cell antigens Esat6/Cfp10 from *Mtb* form tight 1:1 complexes(Renshaw, Panagiotidou et al. 2002; Sacco, Covarrubias et al. 2007)_ENREF_16). Ntrp-HadB/CfplO-Ctrp and Ntrp-Esat6/HadD-Ctrp pairs were used as negative controls. Images were taken after a 3-week incubation at 25 °C.

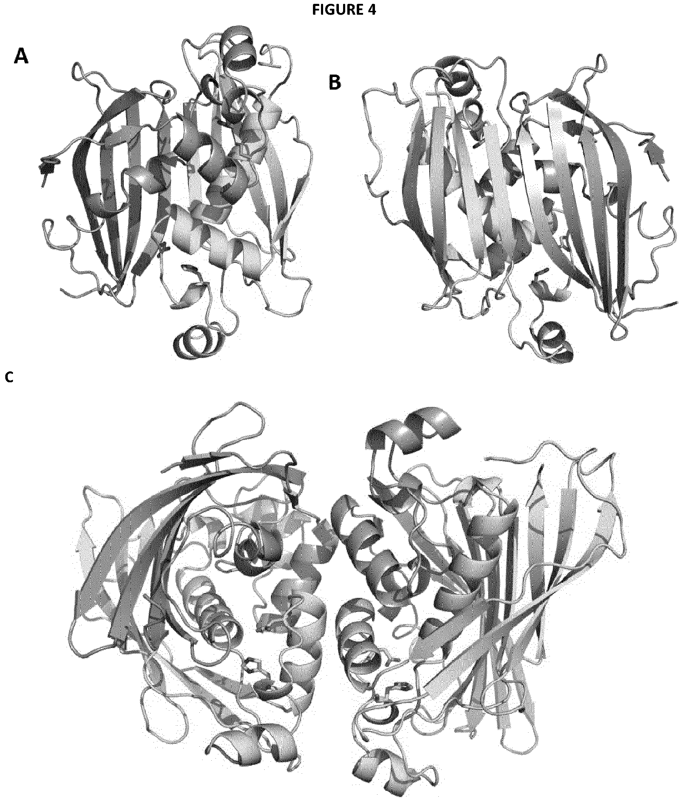

**Figure 4****. Overall structure of HadBD. A.** Heterodimeric structure: front view of the heterodimeric X-ray structure of HadBD. HadB is coloured in cyan and HadD in gray. **B.** Heterodimeric structure: back view of the heterodimeric X-ray structure of HadBD. HadB is coloured in cyan and HadD in gray. Side chains of Asp36 and His41 of HadB (the catalytic residues) as well as Asp37 and His42 of HadD are represented as sticks and coloured by atom type. **C.** Heterotetrameric structure of HadBD as observed in the cristal packing. This model is consistent with a small angle Xray scattering of HadBD in solution. HadB is coloured in cyan and HadD in gray. Side chains of Asp36 and His41 of HadB (the catalytic residues) as well as Asp37 and His42 of HadD are represented as sticks and coloured by atom type

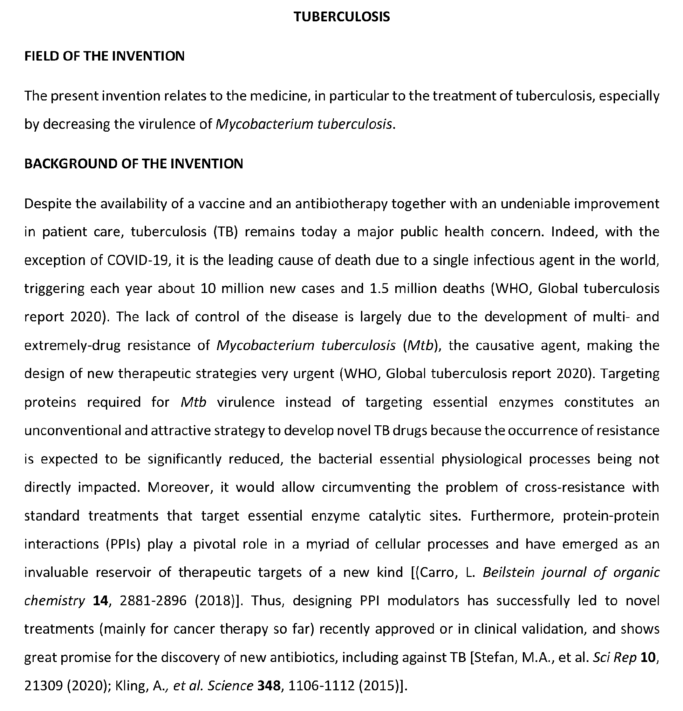

**Figure 5****. HadBD*_{Mtb}* forms an active enzyme with HadB as the catalytic subunit. A.** and **B.** Enzyme activity assays. Kinetic experiments were performed in 100 mM sodium phosphate buffer pH 7.0 in the presence of fixed concentrations of *trans*-2-enoyl-CoA substrate and purified proteins, and monitored by spectrophotometry. Data are means ± average deviations from triplicates. **A.** Assays were done in the presence of 3 µM of either *trans*-2-C12:1-CoA or *trans*-2-C16:1-CoA and HadBD*_{Mtb}* wt. **B.** Assays were done in the presence of 3 µM *trans*-2-C16:1-CoA and the indicated HadBD*_{Mtb}* isoforms.
**Figure 6****. High throughput screening of Fr-PPIChem library based on the whole mycobacteria Split-Trp assay with the strain *M. smegmatis*/Ntrp-hadB/hadD-Ctrp in absence of tryptophan.** The distribution of hit number per average inhibition rate is displayed. On the 10,314 molecules, 137 have been selected with an inhibition rate higher than 70%.

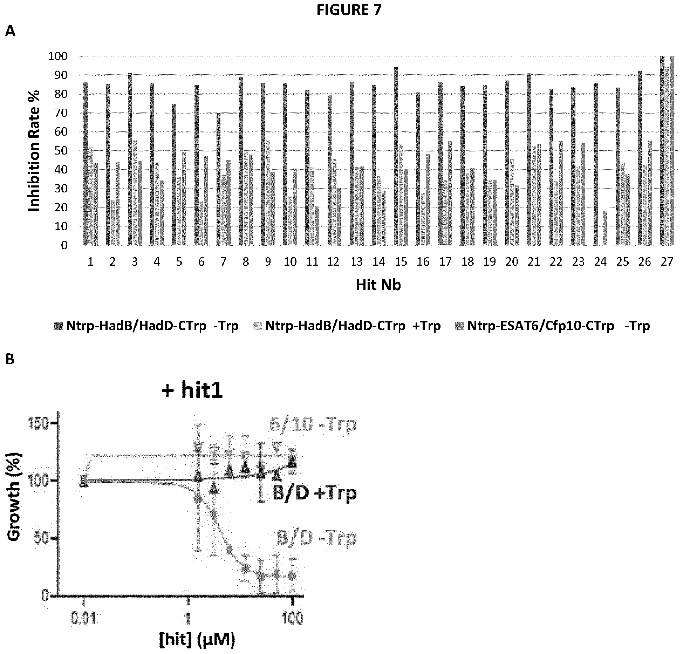

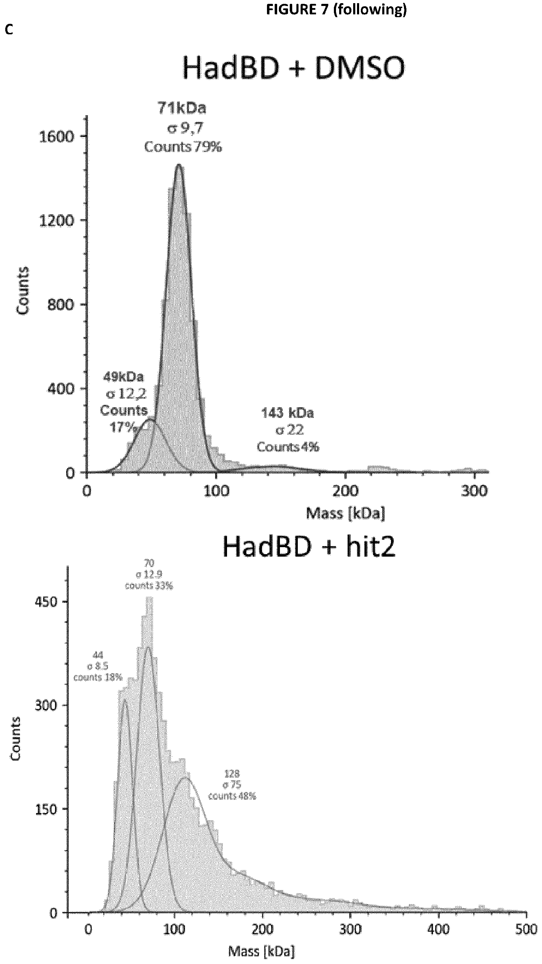

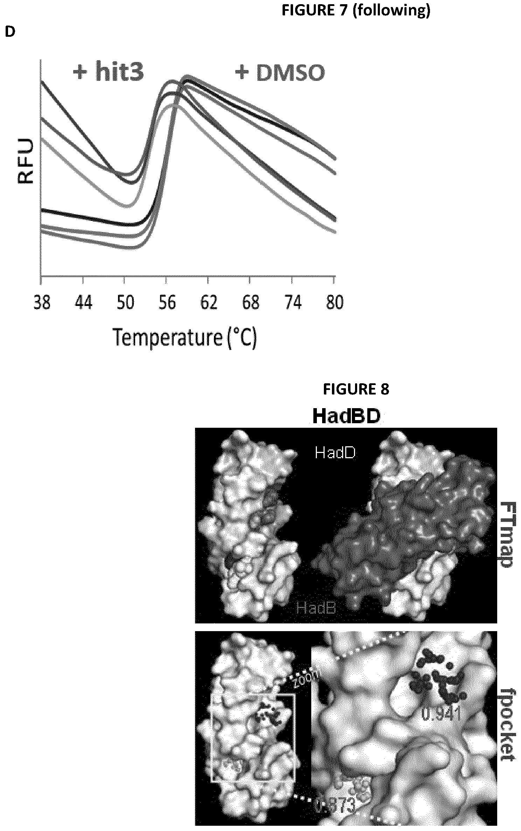

**Figure 7****. Assessment of specificity of the hits. A.** Split-Trp assays with a strain Ntrp-hadB/hadD-Ctrp in presence and absence of tryptophan and with a strain Ntrp-Esat6 / Cfp10-Ctrpin absence of tryptophan with 26 hits previously selected as inhibitors of the interaction between HadB and HadD. **B.** Dose-Response Split-Trp assay with one hit with a strain Ntrp-hadB/hadD-Ctrp in presence and absence of tryptophan and with a strain Ntrp-Esat6 / Cfp10-Ctrp in absence of tryptophan. **C.** Characterization of the effect of one hit on the HadBD heterotetramer by mass photometry. **D.** Characterization of the effect of one hit in the HadBD heterotetramer by differential scanning fluorometry.
**Figure 8. Prediction of hot spots at the protein-protein interface of HadBD.** Surface representation of HadD alone in white or in complex with HadB in skyblue. Top panels: Predictions realized using FTmap (energy based small molecule mapping program). Regions that bind clusters of multiple probe types identify the binding hot spots. The different probes (small molecules) at the protein-protein interfaces are displayed as spheres of distinct colors. Bottom panels: Predictions realized using Fpocket (geometry based binding pocket prediction algorithm). Pockets are highlighted by small spheres. The druggability score (in red), associated using a logistic function, intends to assess the likeliness of a pocket to bind a small drug-like molecule. A score at 0.5 (threshold) indicates that binding can be possible; a score at 1 indicates that binding is very likely.

### DETAILED DESCRIPTION OF THE INVENTION

### Definitions

HadB is a subunit of (3R)-hydroxyacyl-ACP dehydratases of the hydratase 2 family and is encoded by *hadB* (*Rv0636*) gene. Preferably, HadB is from *Mycobacterium tuberculosis (Mtb).* It is described in several databases, namely UniProt ID No I6WYY7; and Gene ID No *Rv0636.* The *Mtb* protein sequence of HadB is disclosed in SEQ ID NO: 1.

HadD is a (3R)-hydroxyacyl dehydratase and is encoded by *hadD* (*Rv0504c*) gene. Preferably, HadD is from *Mycobacterium tuberculosis (Mtb).* It is described in several databases, namely UniProt ID No P9WFK3; and Gene ID No *Rv0504c.* The *Mtb* protein sequence of HadD is disclosed in SEQ ID NO: 2.

### Identification of the inhibitors of the interaction between HadB and HadD proteins

The inventors demonstrated the interactions between HadD and HadB proteins, generating a heterotetramer, and displaying a HAD activity.

The present invention relates to a method for identifying, screening or selecting a molecule capable of preventing the binding of HadD protein to HadB protein of *Mtb* or of inhibiting the interaction between HadB and HadD proteins of *Mtb.* The method comprises determining/measuring the effect of a candidate molecule on the interaction between HadB and HadD proteins of *Mtb,* and selecting the candidate molecule if the interaction between HadB and HadD proteins is decreased or prevented. The method may further comprise a step of contacting the candidate molecule with HadB and HadD proteins of *Mtb* and measuring the effect of the candidate molecule on the interaction of HadB and HadD proteins. Optionally, the candidate molecule could be contacted first with one of HadB and HadD and then contacted with the other. Optionally, the candidate molecule could be contacted with a heterodimer HadBD or a heterotetramer (HadBD₂). The effect of the candidate molecule can be for instance determined by comparing the amount of dimer HadBD or a heterotetramer (HadBD₂) and/or protomer or the stability of the heterotetramer in absence or in presence of the candidate molecule. In particular, the method is *in vitro* or *ex vivo.*

By "inhibit", "decrease" or "prevent" with respect to the interaction between HadB and HadD proteins, it is intended that the interaction is decreased in the presence of a molecule by at least 50, 60, 70, 80 or 90% in comparison of the interaction observed in absence of the molecule. The effect of the molecule on the interaction between HadB and HadD proteins of *Mtb* is determining by measuring the interaction between HadB and HadD proteins in absence and in presence of the tested molecule and by comparing the interaction between HadB and HadD proteins. More specifically, when the interaction between HadB and HadD proteins is assessed by the whole mycobacteriaSplit-Trp assay with a mycobacterial Trp-auxotrophic strain, by "inhibit", "decrease" or "prevent", it is intended that the growth of the Trp-auxotrophic strain is decreased in the presence of a molecule by at least 50, 60, 70, 80 or 90% in comparison of the growth observed in absence of the molecule. More specifically, the effect of the molecule on the interaction between HadB and HadD proteins of *Mtb* is determining by measuring the growth of M. *smegmatis* Ntrp-HadB/HadD-Ctrp in absence and in presence of the tested molecule.

In order to determine the effect of a candidate molecule on the interaction between HadB and HadD proteins, any technology known by the person skilled in the art can be carried out, in particular any method suitable for determining protein-protein interactions. The interaction between HadB and HadD proteins can be measured directly or indirectly.

In a particular aspect, the candidate molecule inhibits the interaction between HadB and HadD proteins and prevents the formation of the heterodimer HadBD. In another aspect, the candidate molecule inhibits the formation of the heterotetramer HadBD₂.

The impact of the molecules on the quaternary structure of the complex, especially HadBD, can be determined by diverse techniques well-known by the person skilled in the art and including non-exhaustively native mass spectrometry (nMS) and mass photometry (MP) (Soltermann, 2021, Phys Chem Chem Phys, 23(31):16488-16500; Erb et al, 2021, Multiprotein Complexes. Method Mol Biol, 2247, 173-191; Marcoux et al, 2013, Proc Natl Acad Sci, 110, 9704-9709), isothermal titration calorimetry, differential scanning fluorimetry (DSF), size exclusion chromatography coupled with multiangle light scattering (SEC-MALS) (Soltermann, 2021, Phys Chem Chem Phys, 23(31):16488-16500), as well as small angle X-ray scattering (SAXS) (Guillet et al, 2019, Nature Comm, 10, 782; Guillet et al, 2016, J Biol Chem, 291, 7973-7989), Hydrogen Deuterium eXchange Mass Spectrometry (HDX-MS) (Guillet et al, 2019, Nature Comm, 10, 782) and X-ray crystallography (Maveyraud & Mourrey, 2020, Molecules, 25, 1030).

Optionally, the method of the present disclosure may comprise a preliminary step for selecting the molecule(s) capable of binding to HadB protein or HadD protein.

The amino acid residues at the HadBD interface are highly conserved during evolution and mutate more slowly than residues at non-binding surfaces. In each heterodimer, the β-strands of both protomers combine to form a 10-stranded antiparallel β-sheet wrapping around two central α-helices, forming a double hot-dog-like fold. The total buried surface at each heterodimer interface is 3836 A². Importantly, the presence of hot spots was predicted at these interfaces in HadBD by energy-based FTmap program and geometry-based Fpocket software. Furthermore, good druggability scores as calculated by Fpocket were obtained for two large pockets at the protein-protein surface of HadD protein in HadBD complex. These data altogether suggest that HadBD has a druggable protein-protein interface. More particularly, a score of 0.5 indicates that binding is possible whereas a score of 1 indicates that binding is very likely. The two identified pockets have a score of 0.941 and 0.873 (see Example 3).

The hot spots of such protein-protein interaction include the residues F21, D37, H39, T41, H42, A51, Y53, V57, A58, P59, L60, T61, F62, L63, A64, 165, A66, G67, R68, R69, V70, Q71, L72, 174, F75, N79, 180, P81, 182, N83, 184, V87, H89, Q92, F94, F96, R98, P99, 1100, F108, T110, 1126, R127, S128, V130, V138, V139, S141, V142, V143, M145, A150, H151 of HadD of *Mtb,* the positions being as indicated in SEQ ID NO: 2. More specifically, the interaction hot spots can form pockets and the following set of pockets have been identified involving residues, as indicated in SEQ ID NO: 2:
- T61, L63, A64, G67, R68, V70, Q71, L72, H89, F75, 184, V87, H89, Q92, F94, F108, S128, V130, S141, V143 and M145;
- D37, H39, T41, H42, A51, Y53, V57, A58, P59, L60, F62, L63, F94, F96, R98, P99, 1100, V138 and V139;
- L63, A64, 165, G67, R68, V70, Q71, H89, Q92, F94, F108, T110, 1126, R127, S128, V130, S141, V142 and V143;
- R68, Q71, L72, 174, F75, 184, V87, H89, V143 and M145;
- D37, H39, T41, H42, A51, Y53, V57, A58, P59 and L60;
- F21, 165, A66, G67, R68 and R69;
- H42, V57, A58, P59, L60, F96, P99 and 1100;
- V57, A58, P59, L60, T61, F62, L63, F96, R98, P99 and 1100;
- L63, A64, G67, R69, Q71, H89, Q92, V143 and M145;
- F75, N79, 180, P81, 182, N83, 184, A150 and H151.

Then, the molecule inhibiting the interaction between HadB and HadD proteins of *Mtb* can be designed and selected to bind HadD at one of the above defined pocket and/or to bind one or several residues of one of the above defined pockets. The molecules can be identified by well-known *in silico* docking techniques (e.g., Agamah et al, Brief Bioinform. 2020;21(5):1663-1675; and online ClusPro docking system of Boston University, https://cluspro.bu.edu/).

Alternatively, the interaction between HadB and HadD proteins can be measured indirectly. More specifically, the inventors developed a screening method based on mixed target-based whole-cell assays, in particular an assay called Split-protein sensor, especially Split-Trp. This strategy allows to discover molecules that i) pass through the permeability barrier of the mycobacterial envelope, ii) are not quickly degraded or eliminated via efflux by bacteria, and iii) directly inhibit protein-protein interaction with a target complex in its native state and physiological environment.

The screening assay is carried out in a Mycobacterium having an auxotrophy, in particular a tryptophane auxotrophic Mycobacterium (i.e. inability to synthesize tryptophane by its own). In a specific aspect, the tryptophane auxotrophic Mycobacterium is a mycobacterial mutant having a deletion or inactivation of the gene *hisA.* The mycobacterium can be for instance *Mycobacterium smegmatis* or *Mycobacterium tuberculosis (Mtb),* preferably *Mycobacterium smegmatis.* More specifically, *Mycobacterium smegmatis* could be used in a high throughput screening and *Mycobacterium tuberculosis* could rather use in a confirmation screening.

More particularly, the Split-Trp relies on the reconstitution of an active Trplp enzyme, especially a Saccharomyces cerevisiae Trplp protein, only if Had proteins interact with each other (i.e., HadB and HadD proteins of *Mtb).* If the interaction occurs, then the tryptophan auxotrophic strain grows on media without tryptophan. This assay has been disclosed in the following articles: O'Hare, et al. 2008, J Microbiol Methods, 73, 79-84; Sacco et al, 2007, Proc Natl Acad Sci U S A 104(37): 14628-14633; Chao et al, 2015, Biochemical and Biophysical Research Communications 458(2): 369-374.

Then, in a particular aspect, the present invention relates to a mycobacterial tryptophan auxotrophic strain comprising an expression cassette encoding a HadB protein of *Mtb* fused to the Ntrp fragment of *Saccharomyces cerevisiae* Trplp protein and an expression cassette encoding a HadD protein *of Mtb* fused to the Ctrp fragment of *Saccharomyces cerevisiae* Trplp protein, thereby providing a mycobacterial tryptophan auxotrophic strain expressing a fusion protein comprising HadB protein of *Mtb* and Ntrp fragment of Trplp protein and a fusion protein comprising HadD protein of *Mtb* and Ctrp fragment of Trplp protein (Ntrp-hadB/hadD-Ctrp). More specifically, the fusion protein comprises the Ntrp fragment of Trplp protein fused to the N terminal end of HadB protein of *Mtb.* For instance, an example of sequence for such a fusion protein is provided in SEQ ID NO: 3. In addition, the fusion protein comprises the Ctrp fragment of Trplp protein fused to the C terminal end of HadD protein of *Mtb.* For instance, an example of sequence for such a fusion protein is provided in SEQ ID NO: 4. Surprisingly, the inventors observed that, when a fusion protein of HadD protein of *Mtb* and Ntrp fragment of Trplp protein and a fusion protein of HadB protein of *Mtb* and Ctrp fragment of Trplp protein are used, a lower growth or an absence of growth of the bacterial strain in observed in absence of tryptophan.

Preferably, the expression cassette(s) of the present invention is/are under the control of an inducible promoter such as an inducible acetamidase promoter.

The present invention relates to a kit for screening methods comprising such a mycobacterial tryptophan auxotrophic strain as disclosed herein and/or vector(s) comprising expression cassette encoding the fusion proteins.

Accordingly, the screening methods or kits may comprise one or several controls, in particular a negative control using a combination of proteins known for the absence of interaction fused to the Ntrp fragment and the Ctrp fragment of Trplp protein and/or a control of target specificity using a combination of proteins known for the presence of interaction and distinct from the target proteins fused to the Ntrp fragment and the Ctrp fragment of Trplp protein, for instance:
a. a control of target specificity with a mycobacterial tryptophan auxotrophic strain expressing a fusion protein comprising the Ntrp fragment of Trplp protein of *Saccharomyces cerevisiae* fused to Esat6 and a fusion protein comprising the Ctrp fragment of Trplp protein of *Saccharomyces cerevisiae* fused to Cfp10 (Ntrp-Esat6 / CfplO-Ctrp); an example of sequence for such fusion proteins is provided in SEQ ID NOs: 5 and 6;
b. a negative control with a mycobacterial tryptophan auxotrophic strain expressing a fusion protein comprising the Ntrp fragment of Trplp protein of *Saccharomyces cerevisiae* fused to HadB protein of *Mtb* and a fusion protein comprising the Ctrp fragment of Trplp protein of *Saccharomyces cerevisiae* fused to Cfp10 (Ntrp-hadB/ CfplO-Ctrp);
c. a negative control with a mycobacterial tryptophan auxotrophic strain expressing a fusion protein comprising the Ntrp fragment of Trplp protein of *Saccharomyces cerevisiae* fused to Esat6 and a fusion protein comprising the Ctrp fragment of Trplp protein of *Saccharomyces cerevisiae* fused to HadD of *Mtb* (Ntrp-Esat6 / HadD-Ctrp).

In a preferred aspect, the kit or method comprises at least one of the negative control or control of target specificity. Optionally, the kit or method comprises one of the following combinations: items a) and c); items a) and d); items b) and c); items b) and d); or items a), b) and c); items a), b) and d); items a), c) and d); items b), c) and d); or items a), b), c) and d). In a specific aspect, the kit or method comprises items a), b) and c).

Accordingly, the present invention relates to a kit comprising 1) a mycobacterial tryptophan auxotrophic strain comprising an expression cassette encoding a HadB protein of *Mtb* fused to the Ntrp fragment of *Saccharomyces cerevisiae* Trplp protein and an expression cassette encoding a HadD protein of *Mtb* fused to the Ctrp fragment of *Saccharomyces cerevisiae* Trplp protein (Ntrp-hadB/hadD-Ctrp); and 2) a mycobacterial tryptophan auxotrophic strain comprising an expression cassette encoding a fusion protein comprising the Ntrp fragment of *Saccharomyces cerevisiae* Trplp protein fused to Esat6 and an expression cassette encoding a fusion protein comprising the Ctrp fragment of *Saccharomyces cerevisiae* Trplp protein fused to Cfp10 (Ntrp-Esat6 / Cfp10-Ctrp). Optionally, the kit can further comprise a mycobacterial tryptophan auxotrophic strain comprising an expression cassette encoding a fusion protein comprising the Ntrp fragment of *Saccharomyces cerevisiae* Trplp protein fused to HadB protein of *Mtb* and an expression cassette encoding a fusion protein comprising the Ctrp fragment of *Saccharomyces cerevisiae* Trplp protein fused to Cfp10 (Ntrp-hadB/ CfplO-Ctrp); and/or a mycobacterial tryptophan auxotrophic strain comprising an expression cassette encoding a fusion protein comprising the Ntrp fragment of *Saccharomyces cerevisiae* Trplp protein fused to Esat6 and an expression cassette a fusion protein comprising the Ctrp fragment of *Saccharomyces cerevisiae* Trplp protein fused to HadD protein of *Mtb* (Ntrp-Esat6 / HadD-Ctrp). Preferably, the kit comprises the mycobacterial tryptophan auxotrophic strains according to items 1), 2) and 3). Optionally, the kit may comprise mycobacterial tryptophan auxotrophic strains comprising all the described expression cassettes.

The present invention also relates to a kit comprising one or several vectors, e.g., plasmids, comprising an expression cassette encoding a HadB protein of *Mtb* fused to the Ntrp fragment of *Saccharomyces cerevisiae* Trplp protein and an expression cassette encoding a HadD protein *of Mtb* fused to the Ctrp fragment of *Saccharomyces cerevisiae* Trplp protein (Ntrp-hadB/hadD-Ctrp), in particular as disclosed above. The kit may further comprise a mycobacterial tryptophan auxotrophic strain. The kit may further comprise one or several vectors, e.g., plasmids, comprising an expression cassette encoding a fusion protein comprising the Ntrp fragment of *Saccharomyces cerevisiae* Trplp protein fused to Esat6 and an expression cassette encoding a fusion protein comprising the Ctrp fragment of *Saccharomyces cerevisiae* Trplp protein fused to Cfp10 (Ntrp-Esat6 / CfplO-Ctrp); and/or an expression cassette encoding a fusion protein comprising the Ntrp fragment of *Saccharomyces cerevisiae* Trplp protein fused to HadB protein of *Mtb* and an expression cassette encoding a fusion protein comprising the Ctrp fragment of *Saccharomyces cerevisiae* Trplp protein fused to Cfp10 (Ntrp-hadB/ CfplO-Ctrp); and/or an expression cassette encoding a fusion protein comprising the Ntrp fragment of *Saccharomyces cerevisiae* Trplp protein fused to Esat6 and an expression cassette a fusion protein comprising the Ctrp fragment of Trplp protein fused to HadD protein of *Mtb* (Ntrp-Esat6 / HadD-Ctrp). Optionally, the kit may comprise a set of vectors comprising all the described expression cassettes.

The present invention also relates to a method for identifying a molecule inhibiting the interaction between HadB protein *of Mtb* and HadD protein *of Mtb,* the method comprising
- providing a mycobacterial tryptophan auxotrophic strain comprising an expression cassette encoding a HadB protein of *Mtb* fused to the Ntrp fragment of *Saccharomyces cerevisiae* Trplp protein and an expression cassette encoding a HadD protein of *Mtb* fused to the Ctrp fragment of *Saccharomyces cerevisiae* Trplp protein (Ntrp-hadB/hadD-Ctrp);
- contacting said strain with a candidate molecule in absence of tryptophan,
- measuring the growth of said strain in presence and in absence of the candidate molecule; and
- selecting the candidate molecule as inhibitor of the interaction between HadB protein of *Mycobacterium tuberculosis* and HadD protein of *Mycobacterium tuberculosis* if the strain contacted with the candidate molecule has a reduced growth in absence of tryptophan compared to the growth of the strain in absence of the candidate molecule and of tryptophan.

Two growth conditions are tested with the mycobacterial tryptophan auxotrophic bacteria:
1) absence of the candidate molecule and absence of tryptophan (control), and
2) presence of the candidate molecule and absence of tryptophan.

Growth conditions of bacteria such as mycobacteria are well known in the art. Suitable substrate, carbon source, nitrogen source and medium can easily by chosen by the man skilled in the art. In particular, the medium comprises histidine and in addition acetamide if an acetamide inducible promoter is used. The method of the invention may preferably be performed at a temperature suitable for bacterial growth, such as temperatures comprised between 20-40°C, preferably between 25°C and 37°C, and under suitable pH conditions. These conditions can be easily adjusted by the skilled person in the art.

Preferably, the strain growth in absence of tryptophan and in presence of the candidate molecule is reduced by at least 50, 60, 70, 80 or 90% compared to the growth of the strain in absence of the candidate molecule and of tryptophan, more preferably more than 70%. Alternatively or additionally; the strain growth in absence of tryptophan and in presence of the candidate molecule represents 50, 60, 70, 80 or 90%, preferably 70%, of the growth of the strain in absence of the candidate molecule and of tryptophan.

Optionally, the method can be carried out with a range of concentrations of the candidate molecule, so as to determine if a dose response on the strain growth is observed. The candidate molecule is preferably selected if a dose response is observed. For instance, the range of concentrations may comprise 3 concentrations with a 10 fold dilution (e.g., ×1, ×10 and ×100). Serial dilutions of the candidate molecule may particularly be tested, for example 1:1, 1:10, 1:100 or 1:1000 dilutions.

In addition, the method may further include controls to verify the specificity of the molecule towards the target-protein complex.

A first control is the same experiment with the strain (Ntrp-hadB/hadD-Ctrp) but in presence of tryptophan. It allows to check that the candidate molecule does not block an essential pathway for the bacterial survival.

Accordingly, the present invention may relate to a method for identifying a molecule inhibiting the interaction between HadB protein of *Mtb* and HadD protein of *Mtb,* the method comprising
- providing a mycobacterial tryptophan auxotrophic strain comprising an expression cassette encoding a HadB protein of *Mtb* fused to the Ntrp fragment of *Saccharomyces cerevisiae* Trplp protein and an expression cassette encoding a HadD protein of *Mtb* fused to the Ctrp fragment of *Saccharomyces cerevisiae* Trplp protein (Ntrp-hadB/hadD-Ctrp);
- contacting said strain with a candidate molecule in presence and in absence of tryptophan,
- measuring the growth of said strain in presence and in absence of tryptophan; and
- selecting the candidate molecule as inhibitor of the interaction between HadB protein of *Mycobacterium tuberculosis* and HadD protein of *Mycobacterium tuberculosis* if the strain contacted with the candidate molecule has a reduced growth of the strain in absence of tryptophan compared to the growth of the strain in presence of both the candidate molecule and tryptophan.

In other words, the strains contacted with candidate molecules in presence of tryptophane that present a higher or similar growth inhibition of the strain (Ntrp-hadB/hadD-Ctrp) in absence of tryptophan are excluded. Preferably, the growth of this strain in presence of tryptophan is not significantly impacted by the selected candidate molecule.

For instance, four growth conditions could be tested with the mycobacterial tryptophan auxotrophic bacteria:
1) absence of the candidate molecule and absence of tryptophan,
2) absence of the candidate molecule and presence of tryptophan,
3) presence of the candidate molecule and presence of tryptophan, and
4) presence of the candidate molecule and absence of tryptophan.

Other controls are possible. For instance, the method may further comprise an assay with a mycobacterial tryptophan auxotrophic strain comprising an expression cassette encoding a fusion protein comprising the Ntrp fragment of *Saccharomyces cerevisiae* Trplp protein fused to Esat6 and an expression cassette encoding a fusion protein comprising the Ctrp fragment of *Saccharomyces cerevisiae* Trp1p protein fused to Cfp10 (Ntrp-Esat6 / CfplO-Ctrp); the assay comprising contacting said strain with a candidate molecule in absence of tryptophane, measuring the strain growth, and selecting the candidate molecule if the growth inhibition of the strain (Ntrp-hadB/hadD-Ctrp) in absence of tryptophan and in presence of the candidate molecule is higher than the growth inhibition of the strain (Ntrp-Esat6 / CfplO-Ctrp) in presence of the candidate molecule and in absence of tryptophan

In other words, the candidate molecules inducing a higher or similar growth inhibition in absence of tryptophan of the strain of the positive control (Ntrp-Esat6 / CfplO-Ctrp) in comparison to the growth inhibition of the test strain (Ntrp-hadB/hadD-Ctrp) are excluded. This control allows to be sure that the Split-Trp assay works and that the effect of the candidate molecule is specific of the interaction between HadB and HadD proteins.

Optionally, the method may further comprise an assay with a mycobacterial tryptophan auxotrophic strain as negative control, said strain comprising an expression cassette encoding a fusion protein comprising the Ntrp fragment of *Saccharomyces cerevisiae* Trplp protein fused to HadB protein of *Mycobacterium tuberculosis* and an expression cassette encoding a fusion protein comprising the Ctrp fragment of *Saccharomyces cerevisiae* Trplp protein fused to Cfp10 Ntrp-hadB/ Cfp10-Ctrp); or a mycobacterial tryptophan auxotrophic strain comprising an expression cassette encoding a fusion protein comprising the Ntrp fragment of *Saccharomyces cerevisiae* Trplp protein fused to Esat6 and an expression cassette a fusion protein comprising the Ctrp fragment of *Saccharomyces cerevisiae* Trplp protein fused to HadD protein of *Mycobacterium* tuberculosis (Ntrp-Esat6 / HadD-Ctrp), the assay comprising contacting said strain with a candidate molecule in absence of tryptophane, measuring the strain growth, and optionally excluding the candidate molecule if the strain growth is increased in presence of the candidate molecule compared to the strain growth in absence of the candidate molecule.

The method according to the invention may be carried out with high throughput assay by using 96 well plates or 384 well plates and a liquid medium. In addition, the inventors used a MTT colorimetric assay for determining the strain growth with enough reliability and sensitivity. Indeed, the optical density is not accurate enough because the tendency of the strains to aggregate. This colorimetric assay is based on the reduction of tetrazolium salt into formazan (see, Bernas and Dobrucki, Biochimica et Biophysica Acta 1451 (1999) 73-81). Then, MTT (-(4,5-dimethylthiazol-2-yl)-2,5 diphenyltetrazolium bromide) is added in the wells and the absorbance is measured at 570 nm, the absorbance being indicative of the strain growth.

The molecules inhibiting HadB and HadD proteins interaction can be peptides or polypeptides or peptide mimetics, antibodies, fragments or derivatives thereof such as nanobodies, aptamers, Spiegelmers, or chemical compounds. The molecules can be selected by the screening methods known in the art or as detailed above and can be designed by any convenient *in silico* modeling method. In a preferred aspect, the molecule is a chemical compound, especially a compound with a molecular mass <1000 Da.

As used herein, the terms "antibody" and "immunoglobulin" have the same meaning and are used indifferently in the present invention. The term "antibody" refers to immunoglobulin molecules and immunologically active portions of immunoglobulin molecules, i.e., molecules that contain an antigen-binding site that immunospecifically binds an antigen. Antibodies include any kind of antibodies, preferably monoclonal. They can be for instance IgG (immunoglobulin G) or VHH (heavy chain variable domain antibody from camelids). Antibodies fragments or derivatives thereof include Fab, Fab', F(ab')2, scFv, (scFv)2, dAb, complementarity determining region (CDR) fragments, linear antibodies, single-chain antibody molecules, minibodies, diabodies, and multispecific antibodies formed from antibody fragments.

In particular, the antibody inhibiting HadB and HadD proteins interaction can bind either HadB or HadD, preferably to HadD of *Mycobacterium tuberculosis* at one or several amino acid residues of a pocket such as described hereabove.

In a particular aspect, the screening method is carried out with a molecules library, especially a chemical library. More specifically, the library can be selected to be more specific of protein-protein interaction. For instance, Fr-PPIChem is a collection of 10,314 molecules dedicated to the inhibition of protein-protein interactions (https://chembiofrance.cn.cnrs.fr/en/composante/chimiotheque; Bosc et al., 2020, ACS Chem Biol, 15(6):1566-1574). Alternative and less specific chemical libraries are also known such as MolPort (https://www.molport.com), Ambinter (http://www.ambinter.com), Enamine Ltd. (https://enamine.net), OTAVA (https://www.otavachemicals.com), WuXi LabNetwork (https://www.labnetwork.com) and Zinc "All Purchasable" (http://zinc.docking.org).

Accordingly, preferably, the method according to the invention is a high throughput method and the method is carried in parallel with more than 100, 500, 1000, 1500, 5000 candidate molecules.

The method for identifying a molecule inhibiting the interaction between HadB protein of *Mtb* and HadD protein of *Mtb* can further comprise additional assays for assessing the specificity and the potency of the selected candidate molecules.

In addition to the impact on the interaction between HadB and HadD proteins, the molecule can be further tested for the inhibitory potency by measuring the enzymatic activity of the purified HadBD of *Mtb* and/or FAS-II system (Sacco et al, 2007, Proc Natl Acad Sci U S A 104(37): 14628-14633; Marrakchi et al, Microbiology (2000), 146, 289-296) and/or by measuring the production level of keto-mycolic acids in mycobacterial cells (Lefebvre C. et al, 2018, Sci Rep 8(1): 6034; Lefebvre C. et al. 2020, Sci Rep 10(1): 2112).

More particularly, in the example section, a detailed method is described for measuring the enzymatic activity of HadBD (see "Enzyme activity assays" in Materials and Methods section).

Furthermore, the candidate molecule can be tested for its antivirulence potency, in particular in a cellular tuberculosis infection model by measuring the ability of the molecule to prevent *Mtb* multiplication in human macrophages, the *Mtb* natural host cells, in particular using High-Content Screening methodology (Christophe et al, 2010, Future Med Chem, 2(8):1283-1293).

In addition, if the preliminary screening has been carried in *Mycobacterium smegmatis,* the Split-Trp assay can be carried out in *Mycobacterium tuberculosis* to confirm the results of the high throughput screening.

Accordingly, the method may comprise one or several of the following assays:
- the Split-Trp assay as detailed above;
- enzymatic assay, in particular by measuring the enzymatic activity of the purified HadBD of *Mtb* and/or FAS-II system; and/or,
- measuring the impact of the selected candidate molecules on the quaternary structure of HadBD; and/or
- antivirulence assay, in particular in a cellular tuberculosis infection by measuring the ability of the molecule to prevent *Mtb* multiplication in human macrophages.

In a particular aspect, the method may comprise one or several of the following assays:
- the Split-Trp assay with the mycobacterial tryptophan auxotrophic strain (Ntrp-hadB/hadD-Ctrp) in absence of tryptophan;
- the Split-Trp assay with the mycobacterial tryptophan auxotrophic strain (Ntrp-hadB/hadD-Ctrp) in presence and in absence of tryptophan and with a specificity control strain such as (Ntrp-Esat6 / CfplO-Ctrp) in absence of tryptophan, all with a dose-response assay of the candidate molecule; and
- measuring the impact of the selected candidate molecules on the quaternary structure of HadBD, for instance by mass photometry and/or differential scanning fluorimetry.

### Therapeutic use of inhibitors of the interaction between HadB and HadD proteins

As previously mentioned, a molecule inhibiting the interaction between HadB and HadD proteins of *Mtb* is useful for the treatment of tuberculosis, in particular by decreasing the virulence of *Mtb.* In addition, when combined with current treatment of tuberculosis, especially antibiotics, said molecule should increase the susceptibility of *Mtb* to the treatment. Moreover, such a molecule should prevent or delay the occurrence of resistance of *Mtb* to said molecule.

Therefore, the present invention relates to a pharmaceutical composition comprising a molecule inhibiting the interaction between HadB and HadD proteins of *Mtb.* Optionally, the pharmaceutical composition further comprises a pharmaceutically acceptable carrier or excipient.

The pharmaceutical compositions contemplated herein may include a pharmaceutically acceptable carrier in addition to the active ingredient(s). The term "pharmaceutically acceptable carrier" is meant to encompass any carrier (e.g., support, substance, solvent, etc.) which does not interfere with effectiveness of the biological activity of the active ingredient(s) and that is not toxic to the host to which it is administered. For example, for parental administration, the active compounds(s) may be formulated in a unit dosage form for injection in vehicles such as saline, dextrose solution, serum albumin and Ringer's solution.

The pharmaceutical composition can be formulated as solutions in pharmaceutically compatible solvents or as emulsions, suspensions or dispersions in suitable pharmaceutical solvents or vehicle, or as pills, tablets or capsules that contain solid vehicles in a way known in the art. Formulations of the present invention suitable for oral administration may be in the form of discrete units as capsules, sachets, tablets or lozenges, each containing a predetermined amount of the active ingredient; in the form of a powder or granules; in the form of a solution or a suspension in an aqueous liquid or nonaqueous liquid; or in the form of an oil-in-water emulsion or a water-in-oil emulsion. Formulations suitable for parental administration conveniently comprise a sterile oily or aqueous preparation of the active ingredient which is preferably isotonic with the blood of the recipient. Every such formulation can also contain other pharmaceutically compatible and nontoxic auxiliary agents, such as, e.g. stabilizers, antioxidants, binders, dyes, emulsifiers or flavouring substances. The formulations of the present invention comprise an active ingredient in association with a pharmaceutically acceptable carrier therefore and optionally other therapeutic ingredients. The carrier must be "acceptable" in the sense of being compatible with the other ingredients of the formulations and not deleterious to the recipient thereof. The pharmaceutical compositions are advantageously applied by injection or intravenous infusion protein of suitable sterile solutions or as oral dosage by the digestive tract. Methods for the safe and effective administration of most of these chemotherapeutic agents are known to those skilled in the art. In addition, their administration is described in the standard literature.

The form of the pharmaceutical compositions, the route of administration, the dosage and the regimen naturally depend upon the condition to be treated, the severity of the illness, the age, weight, and sex of the patient, etc.

The pharmaceutical or therapeutic compositions of the invention can be formulated for a topical, oral, parenteral, intranasal, intravenous, intramuscular, subcutaneous or intraocular administration and the like.

In addition, the present invention relates to a molecule inhibiting the interaction between HadB and HadD proteins or a pharmaceutical composition comprising said molecule for use as a drug, especially as a drug against *Mtb* or for the treatment of tuberculosis.

Thus, the present invention relates to a molecule inhibiting the interaction between HadB of *Mtb* and HadD of *Mtb* or a pharmaceutical composition comprising said molecule for use in the treatment of tuberculosis, in particular by decreasing the virulence of *Mtb.*

It also relates to the use of a molecule inhibiting the interaction between HadB of *Mtb* and HadD of *Mtb* for the manufacture of a medicine for the treatment of tuberculosis, , in particular by decreasing the virulence of *Mtb.*

It further relates to a method of treatment of tuberculosis in a subject in need thereof comprising administering a therapeutic amount of a molecule inhibiting the interaction between HadB of *Mtb* and HadD of *Mtb.* It also relates to a method for decreasing the virulence of *Mtb* in a subject having a tuberculosis comprising administering a therapeutic amount of a molecule inhibiting the interaction between HadB of *Mtb* and HadD of *Mtb.*

By "therapeutically effective amount", it is meant the quantity of the pharmaceutical composition of the invention which prevents, removes, delay or reduces the deleterious effects of tuberculosis in mammals, including humans, alone or in combination with the other active ingredients of the pharmaceutical composition, kit, product or combined preparation. The "therapeutic effective amount" of the composition will be adapted by those skilled in the art according to the patient, the pathology, the mode of administration, etc.

Whenever within this whole specification "treatment of tuberculosis" or the like is mentioned with reference to the pharmaceutical composition of the invention, there is meant: a) a method for treating tuberculosis, said method comprising administering a pharmaceutical composition of the invention to a subject in need of such treatment; b) the use of a pharmaceutical composition of the invention for the treatment of tuberculosis; c) the use of a pharmaceutical composition of the invention for the manufacture of a medicament for the treatment of tuberculosis; and/or d) a pharmaceutical composition of the invention for use in the treatment of tuberculosis.

Within the context of the invention, the term "treatment" denotes curative, and symptomatic treatment. Pharmaceutical compositions and preparations of the invention can be used in humans being infected by *Mtb.* The pharmaceutical compositions and preparations of the invention will not necessarily cure the patient who has a tuberculosis, but could delay or slow the progression or prevent further progression of the disease, ameliorating thereby the patients' condition. In particular, the pharmaceutical compositions and preparations of the invention reduce the *Mtb* virulence and/or the antibiotic resistance or the development thereof.

The molecule inhibiting the interaction between HadB and HadD proteins can be used in combination with an additional therapeutic agent, for instance an antibiotic, in particular an antibiotic targeting *Mtb* or commonly used in the treatment of tuberculosis.

Therefore, the present invention relates to
- a molecule inhibiting the interaction between HadB and HadD proteins or a pharmaceutical composition comprising this molecule for use in the treatment of tuberculosis in combination with an additional therapeutic agent, for instance an antibiotic, in particular an antibiotic targeting *Mtb* or commonly used in the treatment of tuberculosis;
- an antibiotic targeting *Mtb* or a pharmaceutical composition comprising said antibiotic for use in the treatment of tuberculosis in combination with a molecule inhibiting the interaction between HadB and HadD proteins;
- the use of a molecule inhibiting the interaction between HadB and HadD proteins for the manufacture of a medicine for the treatment of tuberculosis in combination with an additional therapeutic agent, for instance an antibiotic, in particular an antibiotic targeting *Mtb* or commonly used in the treatment of tuberculosis;
- the use of an antibiotic targeting *Mtb* for the manufacture of a medicine for the treatment of tuberculosis in combination with a molecule inhibiting the interaction between HadB and HadD proteins;
- a pharmaceutic composition comprising a molecule inhibiting the interaction between HadB and HadD proteins and an antibiotic, in particular an antibiotic targeting *Mtb* or commonly used in the treatment of tuberculosis, preferably for use in the treatment of tuberculosis;
- a product or kit containing a molecule inhibiting the interaction between HadB and HadD proteins and an antibiotic, in particular an antibiotic targeting *Mtb* or commonly used in the treatment of tuberculosis as a combined preparation for simultaneous, separate or sequential use, in particular in the treatment of tuberculosis;
- a combined preparation which comprises a molecule inhibiting the interaction between HadB and HadD proteins and an antibiotic, in particular an antibiotic targeting *Mtb* or commonly used in the treatment of tuberculosis for simultaneous, separate or sequential use, in particular in the treatment of tuberculosis;
- a method for treating tuberculosis in a subject in need thereof, comprising administering an effective amount of a pharmaceutical composition comprising a) a molecule inhibiting the interaction between HadB and Had, b) an antibiotic, in particular an antibiotic targeting *Mtb* or commonly used in the treatment of tuberculosis, and a pharmaceutically acceptable carrier;
- a method for treating tuberculosis in a subject in need thereof, comprising an effective amount of a pharmaceutical composition comprising a molecule inhibiting the interaction between HadB and Had, and an effective amount of a pharmaceutical composition comprising an antibiotic, in particular an antibiotic targeting *Mtb* or commonly used in the treatment of tuberculosis.

The antibiotic can be selected from the list consisting of streptomycin, 4-aminosalicylic acid, isoniazid, pyrazinamide, cycloserine, kanamycin, ethionamide, ethambutol, capreamycin, rifampicin, bedaquiline, delamanid, pretomanid, linezolid, moxifloxacin, levofloxacin, prothiaxamine, amikacin and any combination thereof such as isoniazid and rifampicin, or pyrazinamide and ethambutol. Preferably, the antibiotic is isoniazid and/or rifampicin.

The therapeutic agent for the treatment of tuberculosis to be used in combination with the molecule inhibiting the interaction between HadB and HadD proteins can be one molecule as disclosed in one of the following patent applications: WO22107167, US2022016075, WO22007372, WO21258013, WO21206787, WO21184339, WO21161084, WO21130732, WO21107876, WO21063914, WO21063915, WO21057190, WO21062316, WO21062318, WO21060933, WO21062319, WO21048342, WO21047525, WO21000297, WO20240272, WO20223439, WO20171636, WO20163673, WO20147504, WO20135569, WO20132603, US2020121632, WO20021252, WO19239382, WO19231271, the disclosure thereof being incorporated herein by reference.

Other therapeutic agents that can be used in combination are the BCL-2 inhibitors such as venetoclax 23 (ABT-199), ABT-263, S55746, or ABT-737, the MCL-1 inhibitors such as S63845, A-1210477, MIM-1, MIK665/S-64315, 483-LM, AZD5991, AMG-176, AMG-397, UMI-77, VU661013, or JKY-5-037

In another aspect, the other therapeutic agents can be a vaccine such BCG (Bacille Calmette-Guérin).

In another aspect, the other therapeutic agents can be bacteriophage or a combination of several bacteriophages, such as phage D29, phage AdephagiaA41A43, phage FionnbharthA47, phage Fred313 cpm- 1, and phage MuddyHRMN0052-I.

### EXAMPLES

### Example 1

### Existence of a dehydratase core within the FAS-II system

Given the structural and functional similarities between HadD and HadA-B-C proteins, the inventors wondered whether HadD, although it is encoded by a distinct chromosomal region, is a stand-alone protein or interacts with other HADs *of Mtb* FAS-II system. From pull-downs combined to proteomics analyses in *M. smegmatis,* the inventors have previously observed that HadD*_{Msm}* (MSMEG_0948) makes highly specific physical interactions with HadAB (Lefebvre et al. 2018, *supra*). HadD*_{Msm}* and HadD*_{Mtb}* (Rv0504c) proteins display a relatively high sequence identity (58%) and the chromosomal region of the corresponding genes is partially conserved between *Mtb* and *M. smegmatis* (Lefebvre et al. 2020, *supra*). Furthermore, they are both involved in dehydratation steps during late FAS-II elongation cycles. Yet, phenotypic analyses of deletion mutants as well as cross-complementation experiments showed that HadD*_{Msm}* and HadD*_{Mtb}* are not strict orthologs and have distinct functions, HadD*_{Msm}* being dedicated to the α- and epoxy-MA biosynthesis and HadD*_{Mtb}* to the keto-MA pathway. Therefore, the inventors decided to examine if HadD of *Mtb* complex (including among others *Mtb* and *M. bovis* species) also interact with other HAD subunits. For safety reasons (linked to BSL3 laboratory), the pull downs could not be operated in *Mtb* and were performed from *M. bovis* BCG (handled in a BSL2 laboratory). This strain is closely related to *Mtb* both at genetic and MA content levels; HadD*_{Mtb}* and HadD*_{BCG}* (BCG_0547c) have 100% sequence identity and M. *bovis* BCG synthesizes two MA types found in *Mtb* (α and keto-MAs). Thus, M. *bovis* BCG was transformed by an expression plasmid carrying the hadABC operon from *Mtb* modified in order to produce HadA fused to an enhanced Green Fluorescent Protein (eGFP) tag at its N-terminus. This bait protein was trapped together with interacting proteins using anti-GFP magnetic beads and, after extensive washes, released in mild conditions. The elution fractions, analyzed by nanoLC-MS/MS after trypsin digestion, displayed as expected a strong enrichment in HadB, the second subunit of HadAB enzyme. Interestingly, they were also reproducibly highly enriched in HadC and HadD (Fig. 1). It is noteworthy that HadC was not detected in HadA-based pull downs in *M. smegmatis.* This suggests the existence of a dehydratase core HadA-HadB-HadC-HadD within the FAS-II system of *Mtb* complex species.

### HadD directly interacts with HadB subunit during heterologous coproduction

Although HadD*_{Mtb}* holds the putative catalytic dyad Asp (D37) and His (H42), its hydratase 2 catalytic motif 'F-x(2)-a-x(2)-**D**-x(2)-P-x-**H**-x(5)-A' is degenerate, whereas HadB*_{Mtb}* bears a strictly conserved hydratase 2 motif '[YF]-x(1,2)-[LIVG]-[STGC]-G-**D**-x-N-P-[LIV]-**H**-x(5)-[AS]' (including the catalytic dyad D36 and His41). Furthermore, HadD*_{Mtb}* sequence is much closer to those of HadA*_{Mtb}* and HadC*_{Mtb}* (with 24-28% identity, compared to 33% between HadA*_{Mtb}* and HadC*_{Mtb}*) than that of HadB*_{Mtb}* (only 10% identity). Therefore, the inventors wondered if HadD*_{Mtb}* associates with HadB*_{Mtb}* to form an heterocomplex such as HadAB and HadBC. To answer this question, they first coproduced HadB*_{Mtb}* and N-terminal polyHis-tagged HadD*_{Mtb}* (H-HadD*_{Mtb}*) proteins in *Escherichia coli.* The total soluble proteins were eluted over a cobalt-based IMAC column, potentially followed by size exclusion chromatography (SEC). SDS-PAGE analysis of the purification fractions indicated that the untagged HadB*_{Mtb}* subunit copurified with H-HadD*_{Mtb}* protein (Fig. 2A). The elution volume during the gel filtration step suggested that the protein complex formed a tetramer in solution (Fig. 2B) and size exclusion chromatography (SEC) experiments validated the tetrameric state of the complex. To determine the stoichiometry of the tetramer, the most concentrated purification fractions collected before and after the SEC step were analyzed by native MS (nMS). The spectra clearly showed the presence of an heterotetramer HadBD₂ at the expected mass of 70.4 kDa (Fig. 2C). The presence of some heterodimer HadBD (at a mass of 35.2 kDa) is most likely due to partial dissociation of the tetramer during the nMS analyses.

Altogether, these experiments demonstrate that, when *hadD* and *hadB* are coexpressed, HadD protein associates with HadB to form a heterotetramer in solution. This is reminiscent of the quaternary structures observed for both HadAB and HadBC enzymes from *Mtb* in solution.

### HadB_{Mtb} and HadD_{Mtb} tighly interact in mycobacterial cells

Yet, the issue of the existence of direct physical interactions between HadB*_{Mtb}* and HadD*_{Mtb}* in the physiological context of mycobacterial cells was still raised. In addition, since HadD*_{Mtb}* holds a degenerate catalytic motif, the possibility of the existence of a stand-alone HadD homo-dimeric or tetrameric enzyme had not been ruled out. To investigate these matters, protein-protein interactions (PPIs) sensing experiments in whole-cells, based on the split-Trp method_ENREF_5 adapted to the non-pathogenous rapidly growing *M. smegmatis* species and validated with *Mtb* HADs were performed. The tryptophan (Trp) auxotrophic *M*. *smegmatis* Δ*hisA* strain was cotransformed with a pair of plasmids, each one encoding a target protein (HadB*_{Mtb}* or HadD*_{Mtb}*) in fusion with either the N-terminal fragment (Ntrp) or the C-terminal fragment (Ctrp) of *Saccharomyces cerevisiae* protein Trplp involved in Trp biosynthesis. If an *in vivo* interaction occurs between the target proteins encoded by the pair of plasmids, Trplp function is restored and the recombinant strain grows without exogenous Trp supply. HadB*_{Mtb}*-HadC*_{Mtb}* dehydratase and Esat6-Cfp10 pathogenicity protein complexes from *Mtb,* whose PPIs were previously validated through this split-Trp method, were taken as positive controls. M. *smegmatis* Δ*hisA* producing the protein pair Ntrp-HadD*_{Mtb}*/HadD*_{Mtb}*-Ctrp did not grow on the medium depleted in Trp, whereas the strain producing the pair Ntrp-HadB*_{Mtb}*/HadD*_{Mtb}*-Ctrp displayed a strong growth on this medium (Fig. 3). This growth was more pronounced than with the dehydratase pair Ntrp-HadB_{Mtb}/HadC_{Mtb}-Ctrp and similar to that observed with the control pair Ntrp-Esat6/Cfp10-Ctrp. The lack of growth observed for the negative controls matching proteins of distinct complexes confirmed the specificity of PPIs within the protein complex of interest. Thus, in full agreement with the above data, these experiments showed the existence of heterotypic interactions between HadD*_{Mtb}* and HadB*_{Mtb}*, which form a tight complex within mycobacterial cells, and the lack of HadD homocomplex formation in mycobacteria.

### The HadD protomer of HadBD exhibits structural particularities

The 3D structure of the evidenced HadBD*_{Mtb}* complex was studied by crystallography. The crystals obtained belong to the hexagonal space group *P*6₅22 (a=b= 96.0 Å, c=143.7 Å) with one heterodimer in the asymmetric unit and a solvent content of 55%. The refined structure comprises residues 2-141 of HadB and 13-151 of HadD. The structure of HadBD is that of a double hot dog very close to that observed for HadAB and HadBC (Fig. 4).

Although Had subunits share the same fold formed of the central five-stranded β-sheet and three α-helices, they differ rather significantly in several respects: the additional 3₁₀ helices (ηA in HadA, ηC in HadA and HadB) and β-strands afore mentioned as well as different lengths of the β-strands and connecting loops due to indel regions. One striking difference is the shorter α3 (referred to as hotdog helix) found in HadD, which makes a single turn - compared to four turns in the case of HadA, HadB, and HadC - followed by a series of hydrogen bonded turns and bends, according to the DSSP code. Interestingly, the series of turns and bends that follow the shorter hotdog α3 helix unique of HadD is one of the least ordered region in this protomer (Figure HADS). Furthermore, HadD possesses an additional weakly ordered segment β4-β5, which is shorter and more ordered in HadA and HadC.

The surface of heterodimerization according to PDBePISA analysis is of 1342 Å², with a ΔG=-11 kcal/mol. In the crystal, two heterodimers are related by a crystallographic 2-fold symmetry axis defining an heterotetramer. In the latter, heterodimers interact mainly via the HadB protomers (buried surface area of 525 A² with ΔG=-5,8 kcal/mol). HadD promoters, on their sides, interact with a buried surface of 569 A², but an overall ΔG of +3,7 kcal/mol that reflects a reduced contribution of HadD interaction compared to HadB interaction to the heterotetramerization. Although heterotetramerization occurs via modest interaction surfaces and ΔG, HadBD is usually observed as a tetramer in solution.

### HadBD_{Mtb} forms an active enzyme where HadB is the catalytic subunit.

From a structural point of view, the formation of HadBD complex would allow its activity by analogy to HadAB and HadBC enzymes. To verify that HadBD*_{Mtb}* forms an enzymatic entity, the putative activity of the purified complex was measured by spectrophotometry. Enzymes belonging to the (*R*)-specific enoyl hydratase/hydroxyacyl dehydratase family preferentially catalyze the hydration reaction when they are isolated from their metabolic machinery. Therefore, their activities are most often studied in the presence of enoyl derivatives *in vitro.* Kinetic activity assays were performed in the presence of *trans*-2-dodecenoyl-CoA (*trans*-2-C12:1-CoA) or *trans*-2-hexadecenoyl-CoA (*trans*-2-C16:1-CoA), which can be used as substrates by both HadAB and HadBC. Data showed that the HadBD*_{Mtb}* complex was active and able to metabolize the *trans*-2-ethylenic function of both substrates (Fig. 5A). Yet, its activity was 10 times higher in the presence of the longest enoyl-CoA. This is consistent with the involvement of HadD in late elongation cycles during mycolic acid biosynthesis, implying the transformation of very long-chain natural molecules.

Previous studies on HadAB highlighted that two catalytic residues, Asp36 and His41, in the hydratase 2 motif of HadB are involved in the dehydration mechanism. Structural alignments revealed a very good matching of these residues with the corresponding Asp37 and His42 residues in HadD. The presence of a putative catalytic dyad on both HadB and HadD subunit opens the question of which is the catalytic protomer. Other residues known to be involved in the stabilization of the anionic intermediate within the HadB catalytic site are Ala57 and Gly59, through their respective main-chain CO and NH group, plus potentially Asn38 through side-chain reorientation once the intermediate is generated. These residues are not all conserved in HadD (Ala58, Leu60, and His39). One may notice that Gly59 is positioned at the N-terminus of the straight hotdog helix in HadB, conferring a positive macrodipole. The corresponding residue in HadD, Leu60, does not have this stabilizing effect as the α3 helix is much shorter, disrupting the addition of amino acid microdipoles. Furthermore, in HadB the catalytic residues that lie within a crevice are easily accessible to the substrate, whereas the accessibility would be much more difficult in HadD as the acyl chain should in that case enter through a very narrow channel. These data altogether suggested that HadB protomer could play the role of catalytic subunit in HadBD complex. These data altogether suggest that HadB protomer could play the role of catalytic subunit in HadBD complex.

To verify this hypothesis, mutant isoforms of HadBD*_{Mtb}*, where the putative catalytic His in HadB (H41) and/or HadD (H42) was replaced by a Phe or a Gln residue by site directed mutagenesis, were heterologously produced and purified in exactly the same conditions as those used for the wild type (wt) HadBD*_{Mtb}* complex (see Material and Methods). Such substitutions are classically used to identify the catalytic His residue in dehydratase domains/proteins and a Phe residue is found in place of the catalytic His in the substrate-binding dehydratase subunit HadC*_{Mtb}*. The catalytic activities of the purified HadBD*_{Mtb}* mutant forms were then compared to that of the wt complex in the presence of *trans*-2-C16:1-CoA, the optimal substrate. Both HadB(H41F)-HadDwt and HadB(H41F)-HadD(H42F) forms displayed no detectable activity, whereas HadBwt-HadD(H42F) and HadBwt-HadD(H42Q) lost only 16 to 22% activity (Fig. 5B). It is noteworthy that the variation of specific activities observed could not be linked to an alteration of the oligomeric state since all mutated HadBD enzymes exhibited the same SEC profile as the wt form. Furthermore, the total loss of activity linked to HadB mutation alone could not reasonably be imputed to a change in protein stability, as shown by the relatively weak melting temperature (Tm) shift during differential scanning fluorimetry experiments. In contrast, the partial reduction of activity of HadD-mutated HadBD forms may be due to a partial loss of protein stability since drastic drops (of 12-13°C) of the Tm were observed.

### Materials and Methods

Pull-downs. For pull-down experiments using eGFP-HadA enzyme as a bait, a *M. bovis* BCG Pasteur (ATCC35734) strain was transformed by the plasmid pKW08::*egfp-hodABC_{Mtb}.* The latter was constructed by cloning the *hadABC* (*Rv0635-Rv0636- Rv0637*) operon of *Mtb* H37Rv (ATCC 27294) in the tetracycline-inducible vector pKW08::*egfp* (kindly provided by Andrzej Dziembowski) ((Williams, et al. 2010, Plasmid 64(2): 69-73; Plocinski, et al. 2014, PLoS ONE 9(3): e91380) between BamH1 and Hindlll sites, downstream of the enhanced Green Fluorescent Protein (eGFP) tag coding sequence followed by the Tobacco Etch Virus (TEV) cleavage site. The control strain used for these experiments was *M*. *bovis* BCG/pKW08::*egfp*. The resulting strains were grown in Middlebrook 7H9 broth (Difco) supplemented with 50 µg/ml hygromycin, 10% (v/v) ADC and 0.2% (v/v) glycerol, and the production of the proteins was increased by adding 20 ng/ml tetracycline (Sigma) during the exponential phase. The affinity purification method used was adapted from a previously published protocol (Lefebvre et al, 2018, Sci Rep 8(1): 6034). Bacteria were centrifuged at 10,000 g for 10 min at 4 °C, resuspended in lysis buffer: 50 mM potassium phosphate buffer pH 7.6, 150 mM NaCl, 2 mM AEBSF (Euromedex), 1.7 mM dodecyl 6-D maltoside, 5 µg/ml DNAse I and 5 µg/ml RNase A, and lysed using a Biorupteur Pico (Diagenode) for 30 cycles (30 sec on, 30 sec off). The lysate was clarified by centrifugation at 13,000 g for 30 min at 4 °C and incubated in the presence of GFP-Trap^{®}_MA magnetic beads (Chromotek) for 3 h at 4 °C under agitation. After four washes with 50 mM Tris buffer pH 7.6, 150 mM NaCl, 0.1% (v,v) Triton X-100, proteins were eluted by cleavage using 10 units AcTEV^{™} protease (Fisher) in 50 mM Tris buffer pH 8.0, 150 mM NaCl, 0.5 mM EDTA, 1 mM DTT, overnight at 4°C.

Proteomics analyses. Sample preparation and nanoLC-MS/MS for proteomic analysis were performed as previously described (Duguet, et al. 2017, Molecular & cellular proteomics : MCP 16(8): 1416-1432). Briefly, after reduction and alkylation of cysteine residues, eluted proteins were concentrated in one band by 12% SDS-PAGE. The proteins were then digested with trypsin (1 µg trypsin/50 µg proteins) overnight at 37 °C. The resulting peptides were extracted from the gel, then dried and finally resuspended in 2% acetonitrile, 0.05% TFA. Peptides were analyzed by nanoLC-MS/MS using an UltiMate 3000 RSLCnano system (Dionex, Amsterdam, The Netherlands; 5 to 50% gradient of 80% acetonitrile, 0.2% formic acid for 60 min at 300 nl/min flow rate) coupled to a Q-ExactivePlus mass spectrometer (ThermoScientific, Bremen, Germany).

Raw MS files were analyzed by MS-angel and Proline sofwares. Data were searched with the MASCOT search engine against M. *bovis* BCG Pasteur 1173P2 entries of the Swissprot-Trembl protein database (last update 2021-04-16, 3891 entries) and a list of potential contaminant sequences provided in Proline. Bioinformatics data analysis was performed as described previously (Duguet et al, 2017, *supra*). The dataset contains mass spectrometry results from the analysis of four biological replicates. The statistical proteomics analysis was performed on the LFQ intensities from the "abundance" columns of Proline table.

Native mass spectrometry. Prior to native MS analysis, HadBD sample at 4.8 mg/ml was desalted in 200 mM ammonium acetate, pH 7 using Micro Bio-Spin devices (Bio-Rad, Marnes-la-Coquette, France). Samples were analyzed on a Synapt G2-Si mass spectrometer (Waters, Manchester, UK) running in sensitivity mode, positive ion mode and coupled to an automated chip-based nano-electrospray source (Triversa Nanomate, Advion Biosciences, Ithaca, NY, USA). The voltage applied to the chip and the cone voltage were set to 1.6 kV and 100 V, respectively. The instrument was calibrated with a 2 mg/ml cesium iodide solution in 50% isopropanol. Raw data were acquired in the 1000-8000 m/z range with MassLynx 4.1 (Waters, Manchester, UK) and deconvoluted with UniDec using the following parameters: *m*/*z* range: 1000-8000 Th; Gaussian smoothing: 100; charge range: 5-30; mass range: 10,000-100,000 Da; sample mass every 10 Da; smooth charge states distributions; use automatic *m*/*z* peak width; smooth nearby points : some; suppress artifacts: none; peak detection range: 200 Da, and peak detection threshold: 0.01.

Protein production and purification. The *Rv0504c* gene from *Mtb* H37Rv (ATCC 27294) was amplified by PCR and the product was ligated into pET-15b vector (Novagen) between *Nde*I and *Bam*HI restriction sites; the absence of mutation was verified by sequencing. The *Rv0636* gene from *Mtb* H37Rv was amplified by PCR and the product was ligated into pCDFDuet vector (Novagen) between *Nco*I and *Bam*HI restriction sites; the absence of mutation was verified by sequencing. Site-directed mutagenesis of the *Rv0504c* and HadD*_{Mtb}* genes were carried out by reassembly PCR using doublestranded DNA vector coding HadB or HadD of *M*. *tuberculosis* (pET-15b::*Rv0504c* and pCDFDuet::*Rv0636* respectively) and sense and antisense primers containing the desired mutation. Mutated DNA was used to transform *E. coli* DH5α competent cells and introduction of the point mutation was confirmed by conventional Sanger sequencing of purified plasmids. For co-production of HadB*_{Mtb}* and N-terminal His₆-tagged HadD*_{Mtb}* wild type (WT) and/or mutant proteins, *E. coli* BL21(DE3) (Novagen) was transformed by both pCDFDuet::*Rv0636* and pET-15b::*Rv0504c* constructs. The resulting strains were grown in a 1 L culture at 37 °C under shaking (180 rpm) in LB supplemented with carbenicillin (50 µg/ml) and streptomycin (50 µg/ml). At an OD₆₀₀ of 0.6, gene expression was induced by addition of IPTG at 0.5 or 1 mM IPTG (WT strain and simple mutants), 0.5 mM (HadBH41F-HadDH42Q) or 0.1 mM (HadBH41F-HadDH42F), and bacteria were grown further at 180 rpm, and at 30°C for 4 h (WT strain) or at 20°C for 15 h (all mutants). After centrifugation, the cell pellet was washed in 25 mM Tris buffer, 150 mM NaCl, pH 8.0 and frozen at -80 °C. The latter was resuspended in 50 mM Tris buffer 300 mM NaCl, pH 8.0, and 5% glycerol(buffer A). After addition of 1 mM AEBSF, 1 µg/ml DNase I, 1 µg/ml RNase A, 100 µg/mL lysozyme, bacteria were lyzed by sonication with a Branson Sonifier Cell Disrupter B15 (four 30-sec cycles, microtip 5, 50% duty cycle) or a cell disruptor Emulsiflex C5 Homogenizer (Avestin) (500-1000 psi, 3 times) . After centrifugation at 25,000 g for 20 min at 4 °C, the supernatant was loaded onto 2 mL TALON^{®} superflow metal affinity resin (Clontech) equilibrated with buffer A plus 10 mM imidazole. After extensive washes at 10 mM then 28 mM imidazole in buffer A, the HadB/H-HadD protein complex was mostly eluted at 200 mM imidazole. The elution fractions containing HadBD (as visualized by SDS-PAGE) were pooled and dialyzed twice on a dialysis membrane (size cut-off at 6-8 kDa) in 50 mM Tris/HCI buffer pH 8.0, 150 mM NaCl and 5% glycerol (buffer B), under stirring for 30 minutes. Then, the supernatant was concentrated by centrifugation at 6,000 g and 4°C on a Vivaspin unit (cut-off at 30 kDa). The concentrated protein solution is loaded onto an analytical Bio-Rad ENrich SEC 650 column equilibrated with buffer B and eluted with the same buffer. The elution fractions containing HadBD (as visualized by SDS-PAGE) were pooled, concentrated as described above and the protein concentration is determined by absorbancy measurement at 280 nm (theoritical molar extinction coefficient of HadBD tetramer: 24410 M⁻¹ cm⁻¹).

Split-Trp experiments in *M. smegmatis.* The split-Trp experiments *in M. smegmatis* were performed based on previously described methods (Sacco et al, 2007, Proc Natl Acad Sci U S A 104(37): 14628-14633; O'Hare et al, 2008, Journal of Microbiological Methods 73(2): 79-84; Chao et al, 2010, The Journal of biological chemistry 285(38): 29239-29246) and modified as follows. Briefly, expression plasmids encoding each of the target proteins (HadB*_{Mtb}* and HadD*_{Mtb}*) or a control protein (HadC*_{Mtb}*) in fusion with either the Ntrp or the Ctrp fragment of *Saccharomyces cerevisiae* Trplp protein were constructed by cloning *Rv0636, Rv0504c* and *Rv0637* genes from *Mtb* H37Rv (ATCC 27294) in pJC10 and pJC11 plasmids (Chao et al, 2010, *supra*) (generously provided by Dr. Yossef Av-Gay), under the control of an inducible acetamidase promoter. A tryptophan auxotrophic *M*. *smegmatis* Δ*hisA* strain (O'Hare et al, 2008, *supra*) (kindly provided by Drs Helen O'Hare and Yossef Av-Gay) was cotransformed with pairs of plasmids encoding Ntrp and Ctrp fusion proteins, and grown for 3 days at 37°C on LB supplemented with hygromycin (100 µg/ml) and apramycin (60 µg/ml). Recombinant *M*. *smegmatis* Δ*hisA* colonies were resuspended in 3 ml of LB plus 0.05% Tween 80 and antibiotics and grown at 37°C with shaking for 48 h; the expression of the genes of interest was induced by adding 0.2% (w/v) acetamide for 6 h before stopping the culture (at an OD₆₀₀ of 1-2). Bacteria from 500-µl aliquots were harvested at 10,000 g, washed twice with 1 ml of water plus 0.05% Tween 80, and resuspended in about 1 ml of the same mixture, ajusting all the samples to the same OD₆₀₀. Five-µl aliquots (dilutions 1, 1/10 and 1/100) of each sample were spotted onto 7H9 agar plates supplemented by 0.2% Gro, 0.2% acetamide, 60 µg/ml histidine, 100 µg/ml hygromycin, 60 µg/ml apramycin and depleted in tryptophan. The same dilutions were spotted onto plates containing 60 µg/ml tryptophan to check growth extent. Plates were incubated at 25°C for 3 weeks. Experiments were performed in triplicate. Additionally, *M. smegmatis* Δ*hisA* strains producing either Ntrp-Esat6/Cfp10-Ctrp (pJC10 and pJC11-based expression plasmids (Chao et al, 2010, *supra*) (provided by Dr. Yossef Av-Gay) or Ntrp-HadB_{Mtb}/HadC_{Mtb}-Ctrp were used as positive controls while two strains producing pairwise combinations of proteins from distinct complexes (Ntrp-HadB*_{Mtb}*/Cfp10*_{Mtb}*-Ctrp and Ntrp-Esat6*_{Mtb}*/HadD*_{Mtb}*-Ctrp) were used as negative controls.

Substrate synthesis and purification. *Trans*-2-dodecenoyl-CoA and *trans*-2-hexadecenoyl-CoA were synthesized using the following procedure based on the mixed anhydride method (Goldman and Vagelos, 1961, J. Biol. Chem. 236: 2620-2623). Briefly, three molar equivalents (with respect to CoA) of the corresponding free carboxylic acid (*trans*-2-dodecenoic or *trans*-2-hexadecenoic acid, Larodan) were dissolved in 100 µl anhydrous *t*-butyl methyl ether (*t*-BME, Sigma-Aldrich) per mg of acid. After addition of 1.2 molar equivalents (with respect to carboxylic acid) of both diisopropyl amine and ethyl chloroformate, the reaction was incubated overnight at room temperature. The formed mixed anhydride was separated from ammonium chloride cristals and concentrated under nitrogen. One molar equivalent (with respect to carboxylic acid) of free CoA trilithium salt (Sigma-Aldrich) was dissolved in a 1:1:10.3 M NaHCO₃:ethanol:ethyl acetate mixture at a 5 mg/ml final concentration then added to the mixed anhydride solution. After a 15 min incubation at room temperature, the reaction was quenched by addition of acetic acid. The *trans*-2-enoyl-CoA produced was washed 3 times by one volume of ethyl acetate. It was then purified by HPLC over a 250 × 8 mm ProntoSIL C18 H column (Bischoff) equilibrated with 30% CH₃OH in 20 mM NaH₂PO₄, using a Ultimate 3000 HPLC (ThermoFischer) apparatus with detection at 260 nm. Elution was performed at 1mL/min flow rate using a multiple-step elution program: a gradient from 30 to 40% CH₃OH in 20 mM NaH₂PO₄ (1%/min) with a plateau, then a raise to 100% CH₃OH in H₂O (12%/min) with a plateau. The nature of purified substrates was confirmed by MALDI-TOF MS analysis (see below).

Enzyme activity assays. Hydratase activity was monitored at 263 nm using a UVmc2 spectrophotometer (SAFAS). Kinetic assays were performed in a quartz cuvette in a total volume of 400 µl at room temperature, in 100 mM sodium phosphate buffer pH 7.0 in the presence of either *trans*-2-C12:1-CoA or *trans*-2-C16:1-CoA. After equilibration of the baseline, reactions were started by adding 40 nM of purified HadBD isoform and measurements were recorded for 1-3 min. The initial reaction rates were determined by linear fitting. Enzyme and substrate concentrations were chosen within a window generating a linear response. At 263 nm wavelength, *trans*-2-enoyl-CoAs display an absorbance shift of 0.67 for a variation of concentration of 100 µM. The average specific activities and deviations were calculated from triplicates; the number of active site per HadBD₂ heterotetramer was set to 2 because data reported here showed the presence of two catalytic subunits (HadB) in the complex. The nature of the products was confirmed by MALDI-TOF MS analysis.

### Example 2: Split-Trp Screening of a chemical library

Next, the inventors carried out a screening of the Fr-PPIChem library (10,314 molecules) with a Split-Trp assay modified to be performed in a high throughput way with a tryptophan auxotrophic M. *smegmatis* Δ*hisA* strain expressing fusion proteins Ntrp-HadB and HadD-Ctrp in absence of tryptophan.

As shown in Figure 6, 137 molecules have been identified as having the capacity of inhibiting the interaction between HadB and HadD proteins of *Mtb* with an inhibition rate of at least 70%.

In addition, control assays have been carried out with a tryptophan auxotrophic *M*. *smegmatis* Δ*hisA* strain expressing fusion proteins Ntrp-HadB and HadD-Ctrp in presence of tryptophan and with a tryptophan auxotrophic *M*. *smegmatis* Δ*hisA* strain expressing fusion proteins Ntrp-Esat6 and Cfp10-Ctrp in absence of tryptophan. The first control allows to exclude candidate molecules that would have an impact on an essential pathway. The second control allows to exclude candidate molecules that would have an effect not specific of the interaction between HadB and HadD proteins of *Mtb.* Results are shown in Figure 7A.

In addition, Figure 7B shows the results of a dose-response assay carried out in the three above conditions with a range of eight concentrations of a candidate molecule: strain (Ntrp-HadB / HadD-Ctrp) in absence (B/D -Trp) and presence of tryptophan (B/D +Trp), and strain (Ntrp-Esat6 / CfplO-Ctrp) in absence of tryptophan (6/10 -Trp). The candidate molecule induces a growth inhibition in the assay (B/D -Trp) but not in the controls (B/D +Trp; 6/10 -Trp).

Figure 7C illustrates the result of a mass photometry assay showing the impact of a candidate molecule on the structure of the heterotetramer HadB/HadD. In presence of the candidate molecule, the relative proportion of heterotetramer (peak at about 71 kDa) strongly decreases in favor of molecular species of high molecular weight (peak labeled"128 kDa"). The latter might correspond to aggregates of HadD and/or HadB proteins that are poorly soluble when alone. These aggregates might result from the dissociation of HadB and HadD subunits.

Finally, Figure 7D shows the results of a DSF analysis in presence of a candidate molecule. Assays were realized in triplicates in the absence (orange curves) or presence (blue curves) of candidate molecule. The candidate molecule induces a shift of the denaturation curve and a significative reduction of the melting temperature (Tm) by 2.7°C of the heterotetramer HadBD, suggesting that the candidate molecule is responsible for a destabilization of the protein complex.

For instance, hit #18 and #22 are the followings:

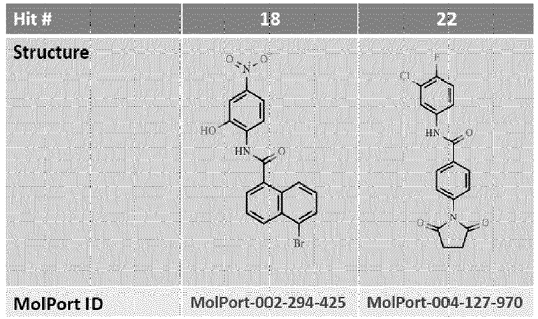

### Materials and Methods

### Screening method in M. smegmatis.

The split-Trp experiments *in M. smegmatis* were performed based on previously described methods (Sacco et al, 2007, Proc Natl Acad Sci U S A 104(37): 14628-14633; O'Hare et al, 2008, Journal of Microbiological Methods 73(2): 79-84; Chao et al, 2010, The Journal of biological chemistry 285(38): 29239-29246) and modified as follows. Briefly, expression plasmids encoding each of the target proteins (HadB*_{Mtb}* and HadD*_{Mtb}*) in fusion with the Ntrp or the Ctrp fragment of *Saccharomyces cerevisiae* Trplp protein, respectively, were constructed by cloning *Rv0636* and *Rv0504c* genes from *Mtb* (ATCC 27294) in pJC10 and pJC11 plasmids (Chao et al, 2010, *supra*) (generously provided by Dr. Yossef Av-Gay), respectively, under the control of an inducible acetamidase promoter. A tryptophan auxotrophic *M. smegmatis* Δ*hisA* strain (O'Hare et al, 2008, *supra*) (kindly provided by Drs Helen O'Hare and Yossef Av-Gay) was co-transformed with pairs of plasmids encoding Ntrp and Ctrp fusion proteins, and grown for 3 days at 37°C in LB supplemented with hygromycin (100 µg/ml) and apramycin (60 µg/ml). Additionally, *M. smegmatis* Δ*hisA* strains producing Ntrp-Esat6/Cfp10-Ctrp protein pair (pJC10 and pJC11-based expression plasmids (Chao et al, 2010, *supra*) provided by Dr. Yossef Av-Gay) was used as control of specificity.

Recombinant *M*. *smegmatis* Δ*hisA* colonies were resuspended in 3 ml of LB plus 0.05% Tween 80 and antibiotics and grown at 37°C with shaking for 48 h. The expression of the genes of interest was induced by adding 0.2% (w/v) acetamide for 6 h before stopping the culture (at an OD₆₀₀ of 1-2). Bacteria from 500-µl aliquots were harvested at 10,000 g, washed twice with 1 ml of water plus 0.05% Tween 80, and resuspended in a volume of water plus 0.05% Tween 80 allowing to adjust all the samples to an OD₆₀₀ of 1.

For 96-well or 384-well microplate assays, a seeding at OD₆₀₀ of 0.001 or 0.1 was used for growing assays in medium with or without tryptophan, respectively. For this purpose, a volume of 0.2 µL of bacterial suspension at OD₆₀₀ of 1 was diluted into 199.8 µL of 7H9 medium also containing by 0.2% glycerol, 0.2% acetamide, 60 µg/ml histidine (medium A) and supplemented by 120 µg/mL tryptophan. A volume of 20 µL of bacterial suspension at OD₆₀₀ of 1 was diluted into 200 µL of medium A (without tryptophan). Then, for both 96-well or 384-well microplate assays, 90 µL of bacterial suspension is dispatched in each well. For assays in the presence of compounds from the chemical library, 1 µL of each molecule of the library diluted in DMSO was added to a well containing bacterial suspension at OD₆₀₀ of 0.1 in medium A, at the desired concentration (final concentration of 10 µM for the primary high throughput screening). In assays without library molecule, the latter was replaced by 1 µL of DMSO. The plates were incubated for 1 week at 30°C. The detection of metabolically active bacteria is performed thanks to the colorimetric MTT assay (see details below). For this purpose, 10 µL of 3-(4,5-dimethylthiazol-2-yl)-2,5-diphenyltetrazolium bromide (or MTT) at 2.5 mg/mL was added in each well, and after a 2h30 incubation at 30°C, 10 µL of SDS 20% was added for bacterial lysis and dissolution of formazan crystals. The day after, the OD570 was measured using a microplate spectrophotometer to evaluate the growth and calculate the growth inhibition rate for each molecule of the library.

The colorimetric MTT assay allows the detection of metabolically active bacteria. It is performed thanks to a method previously described in (Lefebvre et al., 2018, 2020; Slama et al., 2016). Violet color is obtained by the reduction of Tetrazolium salt in metabolically active bacteria. In the case of the split-Trp assay, in a medium without Trp, violet color is obtained only if there is complementation of the Trp biosynthesis in the auxotrophic *M*. *smegmatis* Δ*hisA* strain , i.e. if the Trplp protein is reconstituted. In such MTT assay, bacteria are lysed and release the formazan crystals which are solubilized with SDS. Thus, the amount of formazan produced is proportional to the metabolic activity of the bacteria, which is linked to the bacterial growth. If the growth of all bacteria is inhibited, then the medium will remain yellow in the test. The monitoring of the reduction reaction can be done by measuring the absorbance of the culture at 570 nm, the wavelength of maximum absorbance of formazan. Since the measured absorbance reflects the viability of the bacteria, a threshold is set at an absorbance of 0.6 at 570 nm below which growth is considered inhibited.

The same protocol can be applied to Split-Trp screening experiments in *M*. *tuberculosis.*

### Mass photometry

Purified HadBD heterotetramer was placed in 50 mM Tris/HCI buffer pH 8.0,150 mM NaCl, 5% glycerol, at a concentration of 47 nM, then incubated with excess candidate molecule at 4.7 µM ( 100 equivalents) for 30 min at room temperature. The measurements were carried out using the Refeyn OneMP mass photometer (Refeyn Ltd.). The resulting histograms were fitted to Gaussian distributions using DiscoverMP (Refeyn Ltd.).

### Differential Scanning Fluorometry

HadBD heterotetramer purified to 16 µM final was placed in the presence of Sypro-Orange (10X final - 50 times dilution of the commercial solution at 500X) on a 96-well microplate. Each candidate molecule diluted in DMSO is added to a 96-well microplate at a range of final concentrations of 200, 300, 400 and 500µM (giving a final DMSO concentration of 2, 3, 4 or 5% DMSO respectively), and is incubated in the presence of HadBD for 30 min at room temperature. Controls were carried out without candidate molecule, in the presence of 2, 3, 4 or 5% DMSO alone. The microplate was then subjected to a gradual increase in temperature of 0.3°C/3 seconds, over a range of 20°C to 99.5°C, using a BIO-RAD CFX96TM Real-Time System thermal cycler. C1000TM. Data was recorded and processed with Bio-Rad CFX Manager 3.13 software and fluorescence was measured using the HEX channel. The value of Tm ("melting temperature") was determined by calculating the derivative of the fluorescence curve -d(RFU)/(dT)=f(T).

### Example 3: Predicted hot spots

Predictions of hot spots at the HadB-HadD interface have been carried by using FTmap (https://ftmap.bu.edu/serverhelp.php) and Fpocket (https://bioserv.rpbs.univ-paris-diderot.fr/services/fpocket/).

Figure 8A shows the predictions realized by using FTmap, an energy-based method for the prediction of protein-ligand binding sites. Regions that bind clusters of multiple probe types identify the binding hot spots. The different probes (small molecules) at the protein-protein interfaces are displayed as spheres of distinct colors.

Figure 8B shows the predictions realized by using Fpocket (a geometry based binding pocket prediction algorithm). Pockets are highlighted by small spheres. The druggability score, associated using a logistic function, intends to assess the likeliness of a pocket to bind a small drug-like molecule. A score at 0.5 (threshold) indicates that binding can be possible; a score at 1 indicates that binding is very likely.

**Table A: Prediction by Fpocket**

| **Subunit** | **Pocket number** | **Pocket volume** | **Druggability Score** | **Residues** | |
|---|---|---|---|---|---|
| **HadD** | 1 | 1322.775 | 0.941 | THR 61 | VAL 87 |
| | | | | LEU 63 | HIS 89 |
| | | | | ALA 64 | GLN 92 |
| | | | | GLY 67 | PHE 94 |
| | | | | ARG 68 | PHE 108 |
| | | | | VAL 70 | SER 128 |
| | | | | GLN 71 | VAL 130 |
| | | | | LEU 72 | SER 141 |
| | | | | PHE 75 | VAL 143 |
| | | | | ILE 84 | MET 145 |
| | 2 | 1025.944 | 0.873 | ASP 37 | PHE 62 |
| | | | | HIS 39 | LEU 63 |
| | | | | THR 41 | PHE 94 |
| | | | | HIS 42 | PHE 96 |
| | | | | ALA 51 | ARG 98 |
| | | | | TYR 53 | PRO 99 |
| | | | | VAL 57 | ILE 100 |
| | | | | ALA 58 | VAL 138 |
| | | | | PRO 59 | VAL 139 |
| | | | | LEU 60 | |

**Table B: Prediction by FTmap**

| **Subunit** | **Pocket number** | **Residues** | |
|---|---|---|---|
| **HadD** | 0 | LEU 63 | PHE 108 |
| | | ALA 64 | THR 110 |
| | | ILE 65 | ILE 126 |
| | | GLY 67 | ARG 127 |
| | | ARG 68 | SER 128 |
| | | VAL 70 | VAL 130 |
| | | GLN 71 | SER 141 |
| | | HIS 89 | VAL 142 |
| | | GLN 92 | VAL 143 |
| | | PHE 94 | |
| | 1 | ARG 68 | ILE 84 |
| | | GLN 71 | VAL 87 |
| | | LEU 72 | HIS 89 |
| | | ILE 74 | VAL 143 |
| | | PHE 75 | MET 145 |
| | 2 | ASP 37 | TYR 53 |
| | | HIS 39 | VAL 57 |
| | | THR 41 | ALA 58 |
| | | HIS 42 | PRO 59 |
| | | ALA 51 | LEU 60 |
| | 3 | PHE 21 | GLY 67 |
| | | ILE 65 | ARG 68 |
| | | ALA 66 | ARG 69 |
| | 4 | HIS 42 | LEU 60 |
| | | VAL 57 | PHE 96 |
| | | ALA 58 | PRO 99 |
| | | PRO 59 | ILE 100 |
| | 5 | VAL 57 | LEU 63 |
| | | ALA 58 | PHE 96 |
| | | PRO 59 | ARG 98 |
| | | LEU 60 | PRO 99 |
| | | THR 61 | ILE 100 |
| | | PHE 62 | |
| | 6 | LEU 63 | HIS 89 |
| | | ALA 64 | GLN 92 |
| | | GLY 67 | VAL 143 |
| | | ARG 68 | MET 145 |
| | | GLN 71 | |
| | 7 | PHE 75 | ASN 83 |
| | | ASN 79 | ILE 84 |
| | | ILE 80 | ALA 150 |
| | | PRO 81 | HIS 151 |
| | | ILE 82 | |

### LIST OF SEQUENCES

| Object | SEQ ID NO | Sequence |
|---|---|---|
| HadB of *Mtb* | 1 | MALREFSSVKVGDQLPEKTYPLTRQDLVNYAGVSGDLNPIHWDDEI AKVVGLDTAIAHGMLTMGIGGGYVTSWVGDPGAVTEYNVRFTAVVP VPNDGKGAELVFNGRVKSVDPESKSVTIALTATTGGKKIFGRAIAS AKLA |
| HadD of *Mtb* | 2 | MTVPEEAQTLIGKHYRAPDHFLVGREKIREFAVAVKDDHPTHYSEP DAAAAGYPALVAPLTFLAIAGRRVQLEIFTKFNIPINIARVFHRDQ KFRFHRPILANDKLYFDTYLDSVIESHGTVLAEIRSEVTDAEGKPV VTSVVTMLGEAAHHEADADATVAAIASI |
| Ntrp-HadB | 3 | MPEVVFSVDHSGSGTMYPYDVPDYASSVINFTGSSGPLVKVCGLQS TEAAECALDSDADLLGIICVPNRGGSGSGLKMALREFSSVKVGDQL PEKTYPLTRQDLVNYAGVSGDLNPIHWDDEIAKVVGLDTAIAHGML TMGIGGGYVTSWVGDPGAVTEYNVRFTAVVPVPNDGKGAELVFNGR VKSVDPESKSVTIALTATTGGKKIFGRAIASAKLA |
| HadD-Ctrp | 4 | MALREFSSVKVGDQLPEKTYPLTRQDLVNYAGVSGDLNPIHWDDEI AKVVGLDTAIAHGMLTMGIGGGYVTSWVGDPGAVTEYNVRFTAVVP VPNDGKGAELVFNGRVKSVDPESKSVTIALTATTGGKKIFGRAIAS AKLA MTVPEEAQTLIGKHYRAPDHFLVGREKIREFAVAVKDDHPTHYSEP DAAAAGYPALVAPLTFLAIAGRRVQLEIFTKFNIPINIARVFHRDQ KFRFHRPILANDKLYFDTYLDSVIESHGTVLAEIRSEVTDAEGKPV VTSVVTMLGEAAHHEADADATVAAIASI MPEVVFSVDHSGSGTMYPYDVPDYASSVINFTGSSGPLVKVCGLQS TEAAECALDSDADLLGIICVPNRGGSGSGLKMALREFSSVKVGDQL PEKTYPLTRQDLVNYAGVSGDLNPIHWDDEIAKVVGLDTAIAHGML TMGIGGGYVTSWVGDPGAVTEYNVRFTAVVPVPNDGKGAELVFNGR VKSVDPESKSVTIALTATTGGKKIFGRAIASAKLA MPEVVFSVDHSGSHHHHHHIEGRGSLKMTVPEEAQTLIGKHYRAPD HFLVGREKIREFAVAVKDDHPTHYSEPDAAAAGYPALVAPLTFLAI AGRRVQLEIFTKFNIPINIARVFHRDQKFRFHRPILANDKLYFDTY LDSVIESHGTVLAEIRSEVTDAEGKPVVTSVVTMLGEAAHHEADAD ATVAAIASIIDGSGSGGKRTIDPVIARKISSLVKAYKNSSGTPKYL VGVFRNQPKEDVLALVNDYGIDIVQLHGDESWQEYQEFLGLPVIKR LVFPKDCNILLSAASQKPHSFIPLFDSEAGGTGELLDWNSISDWVG RQESPESLHFMLAGGLTPENVGDALRLNGVIGVDVSGGVETNGVKD SNKIANFVKNAKKGSGKP MPEVVFSVDHSGSGTMYPYDVPDYASSVINFTGSSGPLVKVCGLQS TEAAECALDSDADLLGIICVPNRGGSGSGLKMTEQQWNFAGIEAAA SAIQGNVTSIHSLLDEGKQSLTKLAAAWGGSGSEAYQGVQQKWDAT ATELNNALQNLARTISEAGQAMASTEGNVTGMFA MPEVVFSVDHSGSHHHHHHIEGRGSLKMAEMKTDAATLAQEAGNFE RISGDLKTQIDQVESTAGSLQGQWRGAAGTAAQAAVVRFQEAANKQ KQELDEISTNIRQAGVQYSRADEEQQQALSSQMGFIDGSGSGGKRT IDPVIARKISSLVKAYKNSSGTPKYLVGVFRNQPKEDVLALVNDYG IDIVQLHGDESWQEYQEFLGLPVIKRLVFPKDCNILLSAASQKPHS FIPLFDSEAGGTGELLDWNSISDWVGRQESPESLHFMLAGGLTPEN VGDALRLNGVIGVDVSGGVETNGVKDSNKIANFVKNAKKGSGKP MPEVVFSVDHSGSHHHHHHIEGRGSLKMALKTDIRGMIWRYPDYFI VGREQCREFARAVKCDHPAFFSEEAAADLGYDALVAPLTFVTILAK YVQLDFFRHVDVGMETMQIVQVDQRFVFHKPVLAGDKLWARMDIHS VDERFGADIVVTRNLCTNDDGELVMEAYTTLMGQQGDGSARLKWDK ESGQVIRTAIDGSGSGGKRTIDPVIARKISSLVKAYKNSSGTPKYL VGVFRNQPKEDVLALVNDYGIDIVQLHGDESWQEYQEFLGLPVIKR LVFPKDCNILLSAASQKPHSFIPLFDSEAGGTGELLDWNSISDWVG RQESPESLHFMLAGGLTPENVGDALRLNGVIGVDVSGGVETNGVKD SNKIANFVKNAKKGSGKP |
| Ntrp-Esat6 | 5 | MPEVVFSVDHSGSGTMYPYDVPDYASSVINFTGSSGPLVKVCGLQS TEAAECALDSDADLLGIICVPNRGGSGSGLKMTEQQWNFAGIEAAA SAIQGNVTSIHSLLDEGKQSLTKLAAAWGGSGSEAYQGVQQKWDAT ATELNNALQNLARTISEAGQAMASTEGNVTGMFA |
| Cfp10-Ctrp | 6 | MPEVVFSVDHSGSHHHHHHIEGRGSLKMAEMKTDAATLAQEAGNFE RISGDLKTQIDQVESTAGSLQGQWRGAAGTAAQAAVVRFQEAANKQ KQELDEISTNIRQAGVQYSRADEEQQQALSSQMGFIDGSGSGGKRT IDPVIARKISSLVKAYKNSSGTPKYLVGVFRNQPKEDVLALVNDYG IDIVQLHGDESWQEYQEFLGLPVIKRLVFPKDCNILLSAASQKPHS FIPLFDSEAGGTGELLDWNSISDWVGRQESPESLHFMLAGGLTPEN VGDALRLNGVIGVDVSGGVETNGVKDSNKIANFVKNAKKGSGKP |
| HadC-Ctrp | 7 | MPEVVFSVDHSGSHHHHHHIEGRGSLKMALKTDIRGMIWRYPDYFI VGREQCREFARAVKCDHPAFFSEEAAADLGYDALVAPLTFVTILAK YVQLDFFRHVDVGMETMQIVQVDQRFVFHKPVLAGDKLWARMDIHS VDERFGADIVVTRNLCTNDDGELVMEAYTTLMGQQGDGSARLKWDK ESGQVIRTAIDGSGSGGKRTIDPVIARKISSLVKAYKNSSGTPKYL VGVFRNQPKEDVLALVNDYGIDIVQLHGDESWQEYQEFLGLPVIKR LVFPKDCNILLSAASQKPHSFIPLFDSEAGGTGELLDWNSISDWVG RQESPESLHFMLAGGLTPENVGDALRLNGVIGVDVSGGVETNGVKD SNKIANFVKNAKKGSGKP |
| N-terminal domain from Trplp protein (Ntrp) | 8 | SSVINFTGSSGPLVKVCGLQSTEAAECALDSDADLLGIICVPNR |
| C-terminal domain from Trplp protein (Ctrp) | 9 | KRTIDPVIARKISSLVKAYKNSSGTPKYLVGVFRNQPKEDVLALVN DYGIDIVQLHGDESWQEYQEFLGLPVIKRLVFPKDCNILLSAASQK PHSFIPLFDSEAGGTGELLDWNSISDWVGRQESPESLHFMLAGGLT PENVGDALRLNGVIGVDVSGGVETNGVKDSNKIANFVKNAKKGSGK P |

## Claims

1. A molecule inhibiting the interaction between HadB protein of *Mycobacterium tuberculosis* and HadD protein of *Mycobacterium tuberculosis* or a pharmaceutical composition comprising said molecule for use in the treatment of tuberculosis, in particular by decreasing the *Mycobacterium tuberculosis* virulence.

2. The molecule for use according to claim 1, wherein said molecule is selected from the group consisting of peptides or polypeptides or peptide mimetics, antibodies, fragments or derivatives thereof such as nanobodies, aptamers, Spiegelmers, and chemical compounds.

3. The molecule for use according to claim 1 or 2, wherein the molecule binds to HadD protein of *Mycobacterium tuberculosis* at one or several amino acid residues in position F21, D37, H39, T41, H42, A51, Y53, V57, A58, P59, L60, T61, F62, L63, A64, 165, A66, G67, R68, R69, V70, Q71, L72, 174, F75, N79, 180, P81, 182, N83, 184, V87, H89, Q92, F94, F96, R98, P99, 1100, F108, T110, 1126, R127, S128, V130, V138, V139, S141, V142, V143, M145, A150, H151 of HadD of *Mtb,* the positions being as indicated in SEQ ID NO: 2.

4. The molecule for use according to any one of claims 1-3, wherein the molecule binds to HadD protein of *Mycobacterium tuberculosis* at one or several amino acid residues as defined in the following groups, the positions being as indicated in SEQ ID NO: 2:
- T61, L63, A64, G67, R68, V70, Q71, L72, H89, F75, 184, V87, H89, Q92, F94, F108, S128, V130, S141, V143 and M145;
- D37, H39, T41, H42, A51, Y53, V57, A58, P59, L60, F62, L63, F94, F96, R98, P99, 1100, V138 and V139;
- L63, A64, 165, G67, R68, V70, Q71, H89, Q92, F94, F108, T110, 1126, R127, S128, V130, S141, V142 and V143;
- R68, Q71, L72, 174, F75, 184, V87, H89, V143 and M145;
- D37, H39, T41, H42, A51, Y53, V57, A58, P59 and L60;
- F21, 165, A66, G67, R68 and R69;
- H42, V57, A58, P59, L60, F96, P99 and 1100;
- V57, A58, P59, L60, T61, F62, L63, F96, R98, P99 and 1100;
- L63, A64, G67, R69, Q71, H89, Q92, V143 and M145; and
- F75, N79, 180, P81, 182, N83, 184, A150 and H151.

5. The molecule for use according to any one of claims 1-4, wherein it is to be used in combination with a therapeutic agent treating tuberculosis.

6. The molecule for use according to claim 5, wherein the agent treating tuberculosis is an antibiotic, preferably selected from the group consisting of streptomycin, 4-aminosalicylic acid, isoniazid, pyrazinamide, cycloserine, kanamycin, ethionamide, ethambutol, capreamycin, rifampicin, bedaquiline, delamanid, pretomanid, linezolid, moxifloxacin, levofloxacin, prothiaxamine, amikacin and any combination thereof.

7. The molecule for use according to claim 5, wherein the agent treating tuberculosis is isoniazid and/or rifampicin.

8. A pharmaceutical composition comprising a molecule as defined in any one of claims 1-4, optionally further comprising a therapeutic agent treating tuberculosis, preferably an antibiotic, more specifically selected from the group consisting of streptomycin, 4-aminosalicylic acid, isoniazid, pyrazinamide, cycloserine, kanamycin, ethionamide, ethambutol, capreamycin, rifampicin, bedaquiline, delamanid, pretomanid, linezolid, moxifloxacin, levofloxacin, prothiaxamine, amikacin and any combination thereof, more preferably isoniazid and/or rifampicin.

9. A method for identifying a molecule inhibiting the interaction between HadB protein of *Mycobacterium tuberculosis* and HadD protein of *Mycobacterium tuberculosis,* wherein the method comprises determining the effect of a candidate molecule on the interaction between HadB protein and HadD protein of *Mycobacterium tuberculosis,* and selecting the candidate molecule if the interaction between HadB protein and HadD protein is decreased or prevented.

10. A mycobacterial tryptophan auxotrophic strain comprising an expression cassette encoding a HadB protein of *Mycobacterium tuberculosis* fused to a Ntrp fragment of *Saccharomyces cerevisiae* Trplp protein and an expression cassette encoding a HadD protein of *Mycobacterium tuberculosis* fused a Ctrp fragment of *Saccharomyces cerevisiae* Trplp protein, thereby providing a mycobacterial tryptophan auxotrophic strain expressing a fusion protein of HadB protein of *Mycobacterium tuberculosis* and the Ntrp fragment of Trplp protein of *Saccharomyces cerevisiae* and a fusion protein of HadD protein of *Mycobacterium tuberculosis* and the Ctrp fragment of Trplp protein of *Saccharomyces cerevisiae.*

11. A kit comprising 1) the mycobacterial tryptophan auxotrophic strain according to claim 10, and 2) a mycobacterial tryptophan auxotrophic strain comprising an expression cassette encoding a fusion protein comprising the Ntrp fragment of *Saccharomyces cerevisiae* Trplp protein fused to Esat6 and an expression cassette encoding a fusion protein comprising the Ctrp fragment of *Saccharomyces cerevisiae* Trplp protein fused to Cfp10.

12. A method for identifying a molecule inhibiting the interaction between HadB protein of *Mycobacterium tuberculosis* and HadD protein of *Mycobacterium tuberculosis,* wherein the method comprises
- contacting a strain according to claim 10 with a candidate molecule in absence of tryptophan,
- measuring the growth of said strain in absence of tryptophan; and
- selecting the candidate molecule as inhibitor of the interaction between HadB protein of *Mycobacterium tuberculosis* and HadD protein of *Mycobacterium tuberculosis* if the strain contacted with the candidate molecule has a reduced growth in absence of tryptophan compared to the growth of the strain in absence of the candidate molecule and of tryptophan.

13. The method of claim 12, wherein the method further comprises
- contacting a strain according to claim 10 with a candidate molecule in presence and in absence of tryptophan,
- measuring the growth of said strain in presence and in absence of tryptophan; and
- selecting the candidate molecule as inhibitor of the interaction between HadB protein of *Mycobacterium tuberculosis* and HadD protein of *Mycobacterium tuberculosis* if the strain contacted with the candidate molecule has a reduced growth of the strain in absence of tryptophan compared to the growth of the strain in presence of both the candidate molecule and tryptophan.

14. The method of claim 12 or 13, wherein the method further comprises
- an assay with a mycobacterial tryptophan auxotrophic strain comprising an expression cassette encoding a protein fusion comprising the Ntrp fragment of *Saccharomyces cerevisiae* Trplp protein fused to Esat6 and an expression cassette encoding a fusion protein comprising the Ctrp fragment of *Saccharomyces cerevisiae* Trplp protein fused to Cfp10; the assay comprising contacting said strain with a candidate molecule in absence of tryptophane, measuring the strain growth, and selecting the candidate molecule if the growth inhibition of the strain (Ntrp-HadB/HadD-Ctrp) in absence of tryptophan and in presence of the candidate molecule is higher than the growth inhibition of the strain (Ntrp-Esat6 / CfplO-Ctrp) in presence of the candidate molecule and in absence of tryptophan.

15. The method according to any one of claims 12-14, wherein the method is a high throughput method and the method is carried in parallel with more than 100, 500, 1000, 1500, 5000 candidate molecules.
